# EUROPEAN PATENT APPLICATION

(11) **EP 1 923 052 A1**
(43) Date of publication of application: **21.05.2008**
(21) Application number: 08002116.5
(22) Date of filing: 06.01.2003
(51) Int. Cl.: A61K 9/14, A61K 9/20, A61K 9/48, A61K 31/675

(54) **Novel polymorphs and pseudopolymorphs of risedronate sodium**

(30) Priority: 11.04.2002 US 372465 P; 16.08.2002 US 404174 P; 22.08.2002 US 405668 P
(62) Divisional of application: 03707310.3
(71) Applicant: Teva Pharmaceutical Industries Ltd, 49131 Petah Tiqva (IL)
(72) Inventor: Aronhime, Judith, 76217 Rehovot (IL); Lifshitz-Liron, Revital, Herzlia, 46322 (IL); Kovalevski-Ishai, Eti, Netanya 42255 (IL); Lidor-Hadas, Rami, Kafar-Saba 44242 (IL)
(74) Representative: Russell, Tim

(57) **Abstract**

Provided are novel polymorphs and pseudopolymorphs of risedronate sodium and risedronate disodium, methods for making them, and pharmaceutical compositions containing them.

## Description

### CROSS-REFERENCE TO RELATED APPLICATIONS

This application claims the benefit of provisional applications Serial No. 60/372,465, filed April 11, 2002; Serial No. 60/404,174, filed August 16, 2002, and Serial No. 60/405,668, filed August 22, 2002.

### FIELD OF THE INVENTION

The present invention relates to novel polymorphs and pseudopolymorphs of risedronate sodium and methods of making them. The invention further relates to pharmaceutical compositions containing risedronate sodium in various polymorphic or pseudopolymorphic forms.

### BACKGROUND OF THE INVENTION

Osteoporosis is a disease characterized by a progressive loss of bone mineral. Osteoporosis is also characterized by low bone mass and architectural deterioration of bone tissue leading to enhanced bone fragility and increase in the risk of fracture. The goal of therapy in treatment of osteoporosis is to improve calcium absorption and decrease urinary excretion of calcium thus reversing secondary hyperparathyroidism. Calcium supplements are widely used in managing established osteoporosis but there have been few satisfactory prospective studies of calcium supplementation on bone density or the risk of further fracture. The bisphosphonates, for example etidronate, pamidronate, and risedronate are useful in treating osteoporosis. Risedronate sodium [1-hydroxy-2(3-pyridinyl)ethylidene] bis phosphonic acid monosodium salt), the subject of the present invention, is presently marketed under the tradename Actonel® for treatment of osteoporosis.

Many pharmacologically active substances can exist in more than one crystalline form. The discovery of a new crystalline form of a pharmaceutically useful compound provides an opportunity to improve the performance characteristics of a pharmaceutical product. It enlarges the repertoire of materials that a formulation scientist has available for designing, for example, a pharmaceutical dosage form of a drug with a targeted release profile or other desired characteristic. It is clearly advantageous when this repertoire is enlarged by the discovery of new crystalline forms of a useful compound. For a general review of polymorphs and the pharmaceutical applications of polymorphs consult G.M. Wall, Pharm Manuf. 3, 33 (1986); J.K. Haleblian and W. McCrone, J. Pharm. Sci., 58, 911 (1969); and J.K. Haleblian, J. Pharm. Sci., 64, 1269 (1975), all of which are incorporated herein by reference.

In some cases, foreign molecules, for example solvent molecules, can be regularly incorporated into the crystal structure of a compound. Strictly speaking, such compounds are not true polymorphs and are often referred to as pseudopolymorphs.

The present invention relates to the solid state forms (i.e. polymorphs and pseudopolymorphs) of risedronate sodium that can be prepared by any of the methods herein described. The polymorphs and pseudopolymorphs can be influenced by controlling the conditions under which the salt is obtained in solid form. Solid state physical properties that can differ from one polymorph (or pseudopolymorph) to the next include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch, or tribasic calcium phosphate.

Another important solid state property of a pharmaceutical compound that can depend on crystal structure is its rate of dissolution in aqueous media. The rate of dissolution of an active ingredient in a patient's stomach fluid can have therapeutic consequences because it imposes an upper limit on the rate at which an orally-administered active ingredient can reach the patient's bloodstream. The rate of dissolution is also a consideration in formulating syrups, elixirs and other liquid medicaments. The solid state form of a compound can also affect its behavior on compaction and its storage stability.

These practical physical characteristics can be influenced by the conformation and orientation of molecules in the unit cell, which defines a particular polymorphic form of a substance. The polymorphic form may give rise to thermodynamic properties that are different from those of the amorphous material or another polymorphic (or pseudopolymorphic) form. Thermodynamic properties can be used to distinguish between polymorphs and pseudopolymorphs. Thermodynamic properties that can be used to distinguish between polymorphs can be measured in the laboratory by such techniques as capillary melting point, thermogravimetric analysis (TGA), differential scanning calorimetry (DSC), and differential thermal analysis (DTA).

A particular polymorphic form can also possess distinct spectroscopic properties that may be detectable by, for example, solid state ¹³C NMR spectroscopy and infrared (IR) spectroscopy.

X-ray crystallography on powders (powder diffractometry) can be used to obtain x-ray diffractograms unequivocally distinguish among the crystal structure of different polymorphs and pseudopolymorphs.

United States Patent 6,410,520 ("the '520 patent") describes selective crystallization of risedronate sodium as a monohydrate or hemipentahydrate (pseudopolymorphs). At page 1, the application states, without citation, "It is known in the literature that some bisphonic [sic] acids and their salts are capable of forming hydrates, risedronate sodium exists in three hydration states: mono, hemipenta and anhydrous." The publication also states that the mono and hemipentahydrates were characterized by various means including x-ray diffraction. However, the present inventors have found no such characterization data in the literature for the monohydrate. Also, the present inventors are not aware of any teach in the prior art where a process to make the monohydrate is shown.

In the '520 patent it is written that the monohydrate and the hemipentahydrate are preferred forms and that the hemipentahydrate is the thermodynamically preferred crystalline form under processing conditions based on the observation that the monohydrate crystals convert to the hemipentahydrate form.

The '520 patent also discloses that the monohydrate has a water content from about 5.0% to about 7.1%, more preferably about 5.6% to about 6.5%, and most preferably 5.6%.

The application also discloses that the hemipentahydrate has a water content from about 11.9% to about 13.9%, more preferably about 12.5% to about 13.2%, and most preferably 12.9%. The monohydrate and the hemipentahydrate are further characterized by single crystal X-Ray crystallography, and thermogravimetric analysis, but x-ray results were not disclosed. The present inventors collected x-ray diffraction and TGA data for the forms disclosed in the '520 patent.

In the '520 patent, a process for preparation of the hemipentahydrate form is disclosed. Also, a pharmaceutical composition comprising risedronate from about 50% to about 100% hemipentahydrate and from about 50% to about 0% monohydrate is disclosed.

The '520 patent also discloses methods to selectively make the monohydrate. The present inventors have repeated example 2 of the '520 patent. The product we obtained following the teachings of the '520 patent are a mixture of forms B, A, BB, by x-ray analysis.

### SUMMARY OF THE INVENTION

In one aspect, the present invention provides risedronate sodium having at least one characteristic of form B, which is characterized by x-ray diffraction peaks (reflections) at 2θ values of about 6.0, 14.4, 19.6, 24.9, and 25.4 degrees, or by FTIR absorption bands at about 624, 951, 796, 912, 931, 1046,1105, 1123, 1323, and 1641 cm⁻¹. Form B is a monohydrate as proved by single crystal x-ray analysis.

In another aspect, the present invention relates to pure risedronate sodium form B.

In another aspect, the present invention relates to risedronate sodium form B, stable against transformation to form A.

In a further aspect, the present invention relates to a method of making risedronate sodium having at least one characteristic of form B, especially pure form B, including the step of refluxing a combination of risedronic acid, a sodium base, especially sodium hydroxide, and a mixture of an alcohol, especially ethanol, and water (40-60% water, v/v in alcohol), methanol and water (20%-70%water, v/v, in alcohol), or isopropanol and water (40% - 60% water, v/v, in alcohol). Form B so made is stable against transformation to form A.

In another aspect, the present invention relates to a method of making risedronate sodium having at least one characteristic of form B including the step of exposing risedronate sodium form D to an atmosphere of 80% to 100% RH. The product so made is stable against transformation to form A.

In another aspect, the present invention relates to a method of making risedronate sodium having at least one characteristic of form B including the step of treating risedronate sodium form A with a lower alkanol at a temperature between about room temperature and reflux.

In still another aspect, the present invention provides a method for preparation of risedronate sodium form B by exposing to high relative humidity of 60-100% RH, more preferably 80% RH, risedronate sodium form D for a period of time between 3 and 20 days, more preferably 5-10 days.

In still another aspect, the present invention provides pure risedronate sodium form B. Pure risedronate sodium form B has less than about 2% by weight of form A.

In another aspect, the present invention provides stable risedronate sodium form B. Stable form B does not transform to form A even when exposed to high relative humidity.

In yet another aspect, the present invention relates to risedronate sodium in crystalline form B that is stable against transformation to risedronate sodium in crystalline form A.

In still a further aspect, the present invention relates to risedronate sodium in crystalline form B, stable against transformation to risedronate sodium in crystalline form A when exposed to 75% RH at 40° C for at least three months.

In another aspect, the present invention provides risedronate sodium having at least one characteristic of form B1. Form B1 has characteristic x-ray diffraction peaks (reflections) at 2θ values of about 6.5, 14.7, 21.2, 27.7, and 32.4 degrees 2θ.

In a further aspect, the present invention relates to a method of making risedronate sodium having at least one characteristic of form B1 including the step of refluxing a combination of risedronic acid, at least about two equivalents of a sodium base, and a liquid that is a mixture of an alcohol, especially ethanol, and water (5-25% ethanol, v/v in alcohol). The method can further include one or more cooling steps to room temperature or 5°C or less.

In another aspect, the present invention relates to a method of making risedronate sodium having at least one characteristic of form B1 including the steps of refluxing a suspension of risedronic acid and at least about two equivalents of a sodium base in a mixture of water and ethanol, 50/50 v/v, cooling the suspension to a temperature of about room temperature, further cooling the suspension to a temperature of about 5°C or less, and isolating risedronate sodium having at least one characteristic of form B1 from the suspension.

In another aspect, the present invention relates to a method of making risedronate sodium having at least one characteristic of form B1 including the steps of refluxing a mixture of risedronic acid, at least about two equivalents of a sodium base, and a liquid comprising water and ethanol, 50/50 v/v, and isolating risedronate disodium having at least one characteristic of form B1 from the mixture.

In yet another aspect, the present invention relates to a method of making risedronate sodium form B in admixture with form B1 including the steps of refluxing a combination of risedronic acid and a sodium base in a liquid made-up of between about 5% and about 25%, v/v, ethanol, the remainder essentially water, and isolating the risedronate sodium form the combination.

In still another aspect, the present invention relates to risedronate sodium having at least one characteristic of form BB, which is characterized by x-ray diffraction peaks (reflections) at 2θ values of about 8.5, 9.1, and 9.5, degrees 2θ. X-ray peaks at 5.9, 16.7, 22.0, 24.7, and 28.0 degrees 2θ is a further characteristic of form BB.

In another aspect, the present invention relates to risedronate sodium form BB.

In another aspect, the present invention relates to a method of making risedronate sodium having at least one characteristic of form BB including the steps of providing a solution of risedronate sodium in water at a temperature of about 70°C or more, adding iso-propanol to the solution to obtain a solid-in-liquid suspension, isolating the solid from the suspension, refluxing the isolated solid in suspension in iso-propanol for at least about 10, hours, and isolating risedronate sodium having at least one characteristic of form BB from the suspension.

In yet another aspect, the present invention relates to a method of making risedronate sodium having at least one characteristic of from BB including the steps of exposing risedronate sodium having at least one characteristic of form F to an atmosphere having a relative humidity of at least about 80%.

In yet another aspect, the present invention relates to risedronate sodium having at least one characteristic of form C, which characterized by x-ray diffraction peaks (reflections) at 2θ values of about 5.6, 10.3, 12.9, 26.5, and 30.9 degrees 2θ, or by FTIR absorption bands at about 615, 666, 1089, 1563, and 1615 cm⁻¹.

In another aspect, the present invention relates to a method of making risedronate sodium having at least one characteristic of form C including the step of refluxing a combination of risedronic acid, a sodium base (especially sodium hydroxide), and an alcohol - water mixture, especially an ethanol - water mixture having about 3%, v/v, ethanol, the remainder being water. The method can include one or more cooling steps, for example cooling the mixture to a temperature of 5°C or less.

In a further aspect, the present invention relates to risedronate sodium having at least one characteristic of form D, which can be characterized by x-ray diffraction peaks (reflections) at about 9.9, 17.2, 22.1, 27.9, and 29.2 degrees 2θ, or by FTIR absorption bands at about 697, 807, 854, 955, 1187, 1218, 1576, 1646, and 1719 cm⁻¹.

In yet another aspect, the present invention relates to a method of making risedronate sodium having at least one characteristic of form D including the step of refluxing a combination of risedronic acid, a sodium base (especially sodium hydroxide), and an alcohol or an alcohol/water mixture, especially methanol or a mixture of methanol and water, wherein the mixture is made-up of 1 to about 11%, v/v, water. The method can include one or more cooling steps prior to isolating the risedronate sodium, for example cooling to room temperature or to a temperature of about 5° C or less.

In another aspect, the present invention relates to risedronate sodium having at least one characteristic of form E. Form E is characterized by x-ray diffraction peaks (reflections) at 2θ values of about 8.4, 8.9, 13.6, 27.6, and 27.9 degrees, or by FTIR absorption bands at about 801, 890, 935, 1656, and 1689 cm⁻¹.

In another aspect, the present invention relates to a method of making risedronate sodium having at least one characteristic of form E including the step of refluxing a combination of risedronic acid, a sodium base and an alcohol - water mixture selected from: ethanol containing up to about 80% (v/v) water and methanol containing up to about 80% (v/v) water. The method can include one or more cooling steps before isolating the risedronate sodium, for example cooling to a temperature of about 5° C or less.

In a further aspect, the present invention relates to risedronate sodium having at least one characteristic of form F. Form F can be characterized by x-ray diffraction peaks (reflections) at 2θ values of about 6.6, 8.4, 8.9, 12.2, and 18.6 degrees, or by FTIR absorption bands at about 971, 1133, and 1306 cm⁻¹. Form F is stable against transformation to form A hemipentahydrate when exposed to high relative humidity.

In another aspect, the present invention relates to a method of making form F including the step of heating risedronate sodium forms B and A to a temperature between about 120° and 180° C for about 2 to about 10 hours.

In still another aspect, the present invention relates to risedronate sodium having at least one characteristic of form G. Form G is characterized by x-ray diffraction peaks at 2θ values of about 8.0, 9.9, 12.2, 15.2, and 19.6 degrees, or by FTIR absorption band s at about 724, 871, 1174, and 1285 cm⁻¹.

In another aspect, the present invention relates to a method of making risedronate sodium having at least one characteristic of form G including the step of heating a combination of risedronate forms A and E at a temperature of between about 120° and 180°C. In another aspect, the present invention relates to the just-recited method for making form G wherein the temperature is about 160°C and the time of heating is between about 5 and about 8 hours.

In still a further aspect, the present invention relates to risedronate sodium having at least one characteristic of form H. Form H is characterized by x-ray diffraction peaks (reflections) at 2θ values of about 6.9, 9.8, 10.9, 13.7, 16.0, and 18.0 degrees.

In another aspect, the present invention relates to a method of making risedronate sodium having at least one characteristic of form H including the step of exposing risedronate sodium form C to high relative humidity (> 60% RH) for about 3 to about 20 days. In still another aspect, the present invention relates to the just-recited method for making form H wherein the percent relative humidity is >80% and the risedronate sodium is exposed for a period of about 7 to about 14 days.

In another aspect, the invention provides a method for preparation of risedronate sodium having at least one characteristic of form A by combining at reflux temperature risedronic acid and sodium hydroxide in water or water solutions of isopropanol or ethanol (e.g., 20%, v/v, isopropanol and water) to yield product having at least one characteristic of form A, the reaction taking place for a period of at least about 1 hour. The method can further include one or more cooling steps prior to isolating risedronate sodium, for example cooling to a temperature of about 5° C or less.

In yet another aspect, the present invention provides a method for preparation of form A by exposing to high relative humidity of 60-100% RH risedronate sodium form G for a period of time between 3 and 10 days. In another aspect, the present invention provides a method of making form A according to the just-recited method wherein the %RH is greater that about 80% and the exposure is for a time of about 7 days.

In still another aspect, the present invention provides a method for preparation of form A by exposing to high relative humidity of 60-100% RH, more preferrably 80% RH, form E or form G for a period of time between 3 and 10 days, more preferrably 7 days.

In yet a further aspect, the present invention relates to a method of making risedronate sodium having at least one characteristic of form A including the step of treating risedronate sodium with water at a temperature between about room temperature and reflux temperature.

In still a further aspect, the present invention provides a method for preparation of form H by exposing to high relative humidity of 60-100% RH, more preferrably 80% RH, form C for a period of time between 3 and 20 days, more preferrably 5-10 days.

Another aspect of the present invention is preparation of form A by heating form E at temperatures in the range 30-100°C.

In yet another aspect, the present invention relates to pharmaceutical compositions, suitable for administration to a host in need of treatment for disorders relating to, for example, calcium homeostasis or bone density, containing one or more of forms A, B, B1, BB, C, D, E, F, G, and H of risedronate sodium and at least one pharmaceutically acceptable excipient.

In still another aspect, the present invention provides a pharmaceutical composition containing pure risedronate sodium form B.

In another aspect, the present invention provides a pharmaceutical composition containing risedronate sodium form B, stable against transformation to form A.

In yet another aspect, the present invention provides a pharmaceutical composition containing stable risedronate sodium form B. Upon exposure to 75 % RH at 40°C for a period of about 6 months, less than 40% by weight of the risedronate sodium in this composition transforms to form A.

### BRIEF DESCRIPTION OF THE DRAWINGS

Figure 1 is the x-ray powder diffraction diagram of risedronate sodium form A hemipentahydrate.
Figure 2 is the TGA curve of Risedronate sodium form A hemipentahydrate.

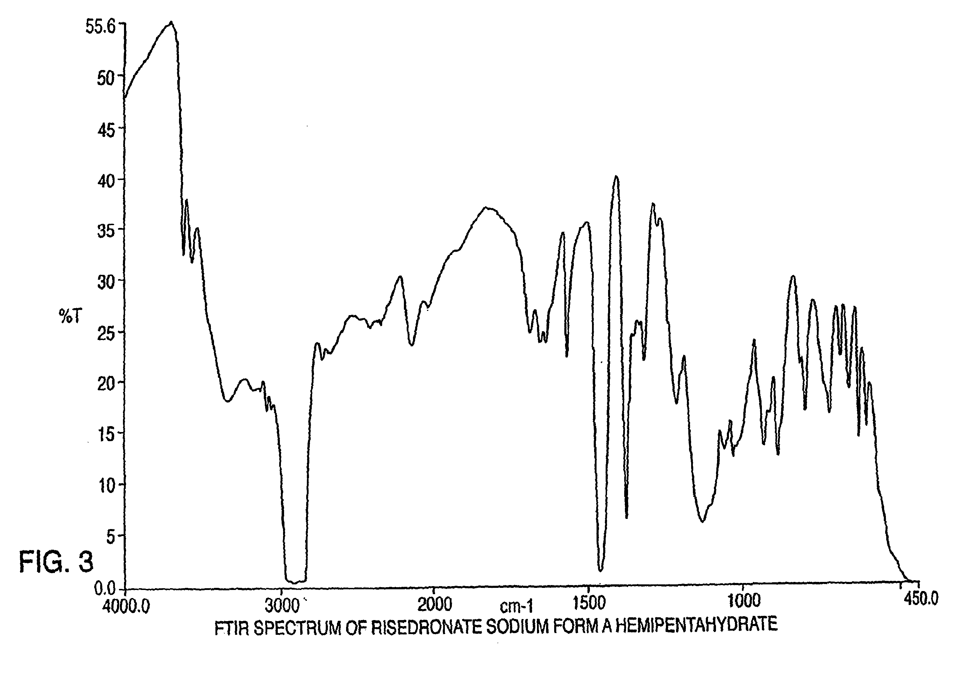

Figure 3 is the FTIR spectrum of Risedronate sodium form A hemipentahydrate.
Figure 4 is the X-Ray powder diffraction of Risedronate sodium form B.

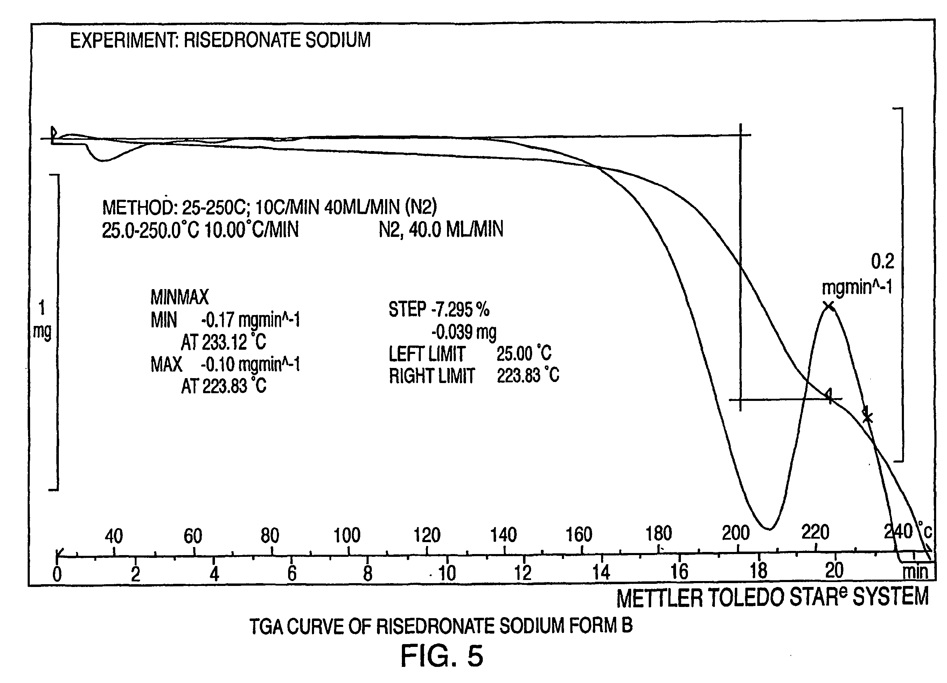

Figure 5 is the TGA curve of Risedronate sodium form B.
Figure 6 is the FTIR spectrum of Risedronate sodium form B.
Figure 7 is the X-Ray powder diffraction of Risedronate sodium form BB.
Figure 8 is the TGA curve of Risedronate sodium form BB.
Figure 9 is the X-Ray powder diffraction of Risedronate sodium form B1.
Figure 10 is the TGA curve of Risedronate sodium form B1.
Figure 11 is the X-Ray powder diffraction of Risedronate sodium form C.
Figure 12 is the TGA curve of Risedronate sodium form C.
Figure 13 is the FTIR spectrum of Risedronate sodium form C.
Figure 14 is the X-Ray powder diffraction of Risedronate sodium form D.
Figure 15 is the TGA curve of Risedronate sodium form D.
Figure 16 is the FTIR spectrum of Risedronate sodium form D.
Figure 17 is the X-Ray powder diffraction of Risedronate sodium form E.

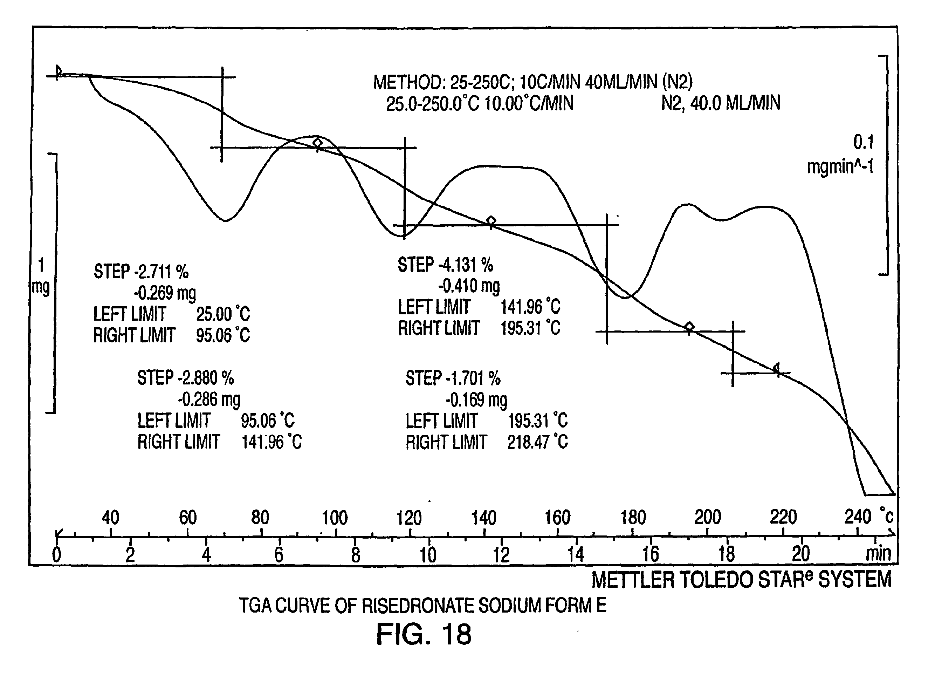

Figure 18 is the TGA curve of Risedronate sodium form E.
Figure 19 is the FTIR spectrum of Risedronate sodium form E.
Figure 20 is the X-Ray powder diffraction of Risedronate sodium form F.
Figure 21 is the TGA curve of Risedronate sodium form F.
Figure 22 is the FTIR spectrum of Risedronate sodium form F.
Figure 23 is the X-Ray powder diffraction of Risedronate sodium form G.
Figure 24 is the TGA curve of Risedronate sodium form G.
Figure 25 is the FTIR spectrum of Risedronate sodium form G.

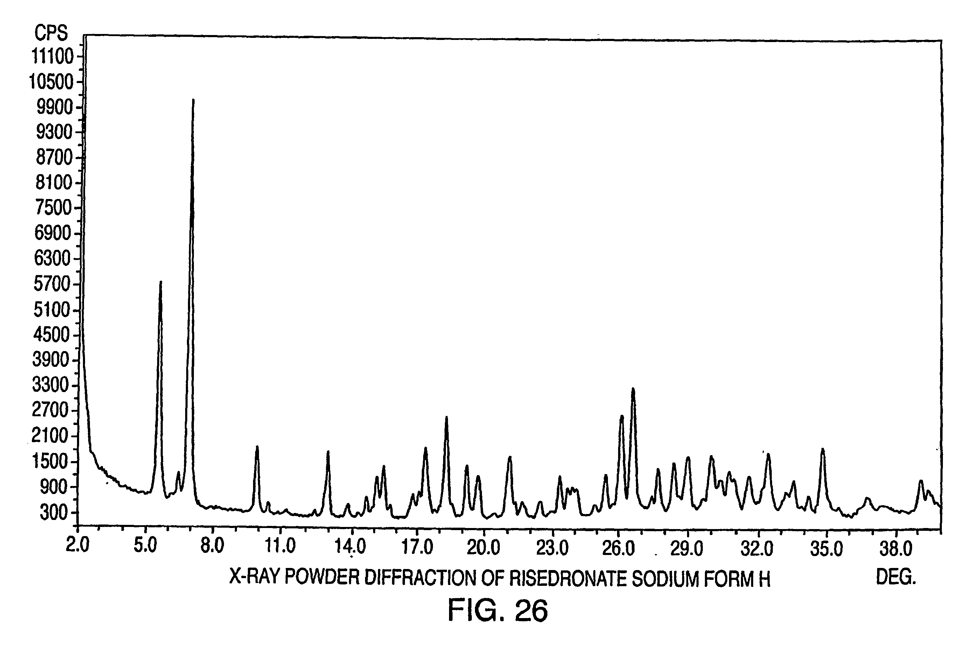

Figure 26 is the FTIR spectrum of risedronate sodium form H.

### DETAILED DESCRIPTION OF THE INVENTION

As used herein, risedronate sodium and sodium risedronate refer to the monosodium salt of risedronic acid, i.e., 1-hydroxy-2(3-pyridinyl)ethylidene bis phosphonic acid monosodium salt. Risedronate sodium has the empirical formula C₇H₁₀NO₇P₂Na.

Unless otherwise required by the context, as used herein risedronate sodium and sodium risedronate do not denote the material in any particular physical state and include amorphous material as well as material in any crystalline form.

As used herein in connection with a measured quantity, the term "about" indicates that variation in the measured quantity as would be expected by the skilled artisan making the measurement or determination and exercising a level of care commensurate with the objective of the measurement and the precision of the measuring apparatus being used.

As used herein, the term sodium base refers to a base having sodium as a cation. Examples of sodium bases include NaOH, Na₂CO₃, and Na HCO₃. NaOH is the preferred sodium base.

As used herein, the term lower alkanol refers to compounds of the general formula ROH, where R is a linear or branched alkyl group having up to 6 carbon atoms.

As used herein in connection with liquids that are mixtures, v/v and volume/volume refer to the ratio of volumes of liquids (e.g. alcohols and water) that are combined to make the liquid. Thus, 50/50, v/v, refers to a mixture made by combining approximately equal volumes of two liquids.

As used herein TGA weight loss is determined by calculating the weight loss over the temperature range up to about 200° by 220°C at the inflection point of the weight loss curve (see Figures).

The abbreviations "RH" and "%RH" have the customary meanings and denote the percent relative humidity of an atmosphere.

The term room temperature refers to a temperature of about 25° C.

X-ray diffraction data described herein was obtained by the powder diffraction method. X-Ray powder diffraction data were obtained using methods known in the art with a SCINTAG powder X-Ray diffractometer model X'TRA equipped with a solid state detector. Copper radiation of 1.5418 Å was used. A round aluminum sample holder with a round zero background quartz plate, with cavity of 25mm x 0.5 mm.

X-ray diffraction analysis can be used to detect and quantify one crystalline form of risedronate sodium in another. Using x-ray diffraction analysis, the presence of about 1% by weight or less of form A in form B can be detected.

Fourier Transform Inrfared spectroscopy (FTIR) is an analytical technique well known in the art employing polychromatic radiation and Fourier transformation of the interferogram obtained. FTIR spectra disclosed herein were recorded for nujol mulls of samples using a Perkin Elmer Spectrum 1 instrument.

Thermogravimetric analysis (TGA) is a technique of thermal analysis well known in the art and measures the change in weight of a sample as a function of temperature. The technique is particularly well suited for measurement of, for example, decomposition and desolvation. TGA results reported herein were obtained using a Mettler TG50. Sample size was between about 6 and about 15 mg. Samples were analyzed at a heating rate of 10°C/min from 25°C to 250C. The oven was purged with nitrogen gas at a flow rate of 40 ml/min. Standard alumina crucibles covered by lids with one hole were used.

As used herein, the phrase "having at least one characteristic of form '#"', where '#' is a letter or letter and Arabic numeral (e.g. form B, form B1, etc.), refers to a crystalline form of risedronate sodium that exhibits at least the characteristic x-ray peaks or the characteristic FTIR absorption bands of form '#'.

As used herein in connection with a crystalline form (polymorph or pseudopolymorph) of risedronate sodium, the phrase "does not substantially convert to the hemipentahydrate form" means that not more than about 20% of the polymorph or pseudopolymorph converts or rearranges to hemipentahydrate (risedronate sodium form A).

As used herein in connection with risedronate sodium form B, the term pure denotes form B substantially free of risedronate sodium hemipentahydrate form A. Substantially free means less than about 1 % on a weight basis as determined by, for example, x-ray diffraction analysis.

As used herein in connection with form B, stable against transformation to form A means that not more than about 20% of form B transforms to form A under the specified conditions. When made according to the preferred embodiments of the present invention, less than 20% of form B transforms to form A upon exposure to a RH of 75% at 40° C for a period of at least three months.

The stability of crystalline forms of risedronate sodium against transformation to form A is measured by exposing a sample to an atmosphere having a percent relative humidity (RH or %RH) of at least about 50% at a temperature greater than room temperature for a period of time. It is convenient to evaluate the stability of a crystalline form of risedronate sodium against transformation to form A by exposing a sample to an atmosphere having RH of about 75% at a temperature of 40°C for a period of at least 3 months. The experience of the skilled artisan teaches that a pharmaceutical that is stable under these conditions will be stable for at least 2 years at room temperature. Hence, the skilled artisan would anticipate, with reasonable expectation of being correct, that form B stable against transformation to form A when exposed to 40°C/ 75% RH for six months would not substantially convert to form A when stored at room temperature for four years.

Hemipentahydrate reference material was prepared according to the procedure of example 1 in the '520 patent. The hemipentahydrate was denominated by us form A, and was identified as the crystal form present in the commercial tablet ACTONEL.

Form A hemipentahydrate, prepared according to the procedure of example 1 in the '520 patent, has been characterized by X-Ray, FTIR, and TGA.

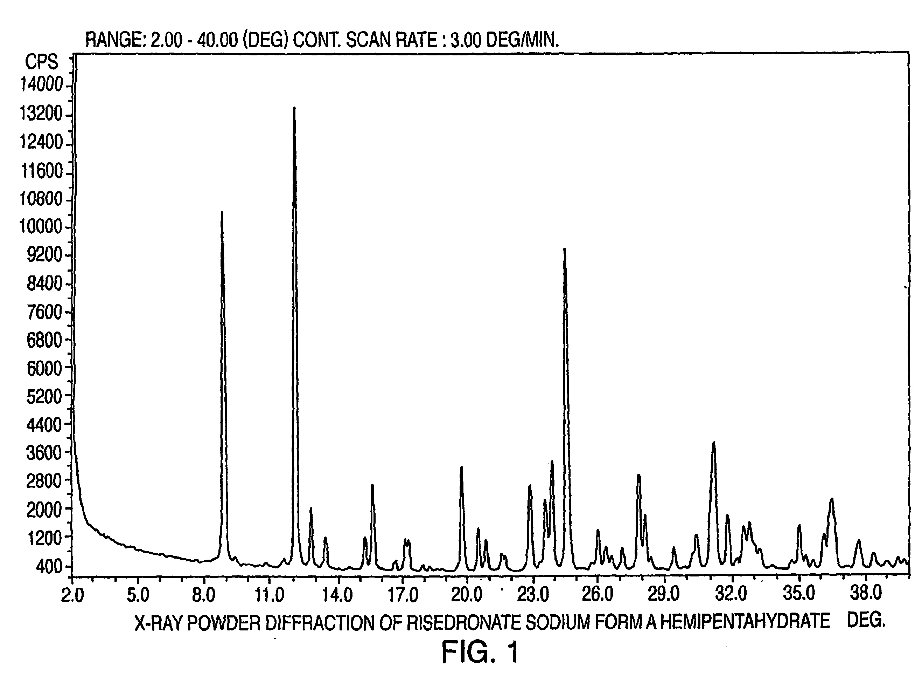

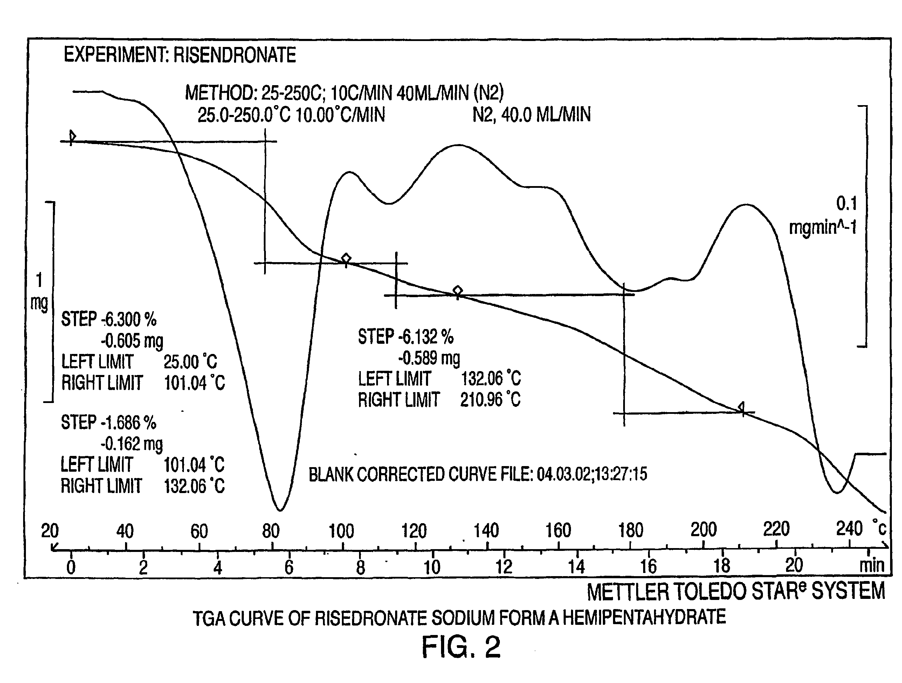

One characteristic of form A is its x-ray diffraction pattern. The x-ray diffraction diagram of form A is shown in Fig.1, and the TGA curve obtained is shown in Fig.2. Form A hemipentahydrate is characterized by x-ray peaks at 8.9, 12.2, 24.6 degrees 2-theta and other peaks at 12.9, 13.5, 15.4, 15.7, 27.8, 28.1, 31.3 degrees 2-theta. The TGA curve shows multiple weight loss steps, for an overall weight loss of 12-14%, which conforms to the value of 11.9-13.9% water content reported in the '520 patent.

Another characteristic of form A is its absorption bands in FTIR spectroscopy. The FTIR spectrum of form A shows characteristic peaks at 800, 889, 935, 1132, 1637, 1657, 1689 cm⁻¹.

The procedure of examples 1 and 2 of the '520 patent for the preparation of the so called monohydrate form was repeated and, in our hands, consistently led to a product that included forms B, BB and A. The X-Ray powder diffraction pattern of form BB differs from that of form A. The TGA thermogram shows multiple weight loss steps, for an overall weight loss of 9.5-10.0%, which does not conform to the value of 5.0-7.1 % for the monohydrate reported in the '520 patent.

In one embodiment, the present invention provides risedronate sodium having the characteristics of form B that was shown to be monohydrate by single crystal x-ray analysis. Form B has a weight loss in TGA of between about 5% and about 8%. The TGA weight loss step of monohydrate form B is larger than the expected based on theoretical water content of 5.6%; presumably due in the fact that the TGA weight loss step also reflects decomposition processes.

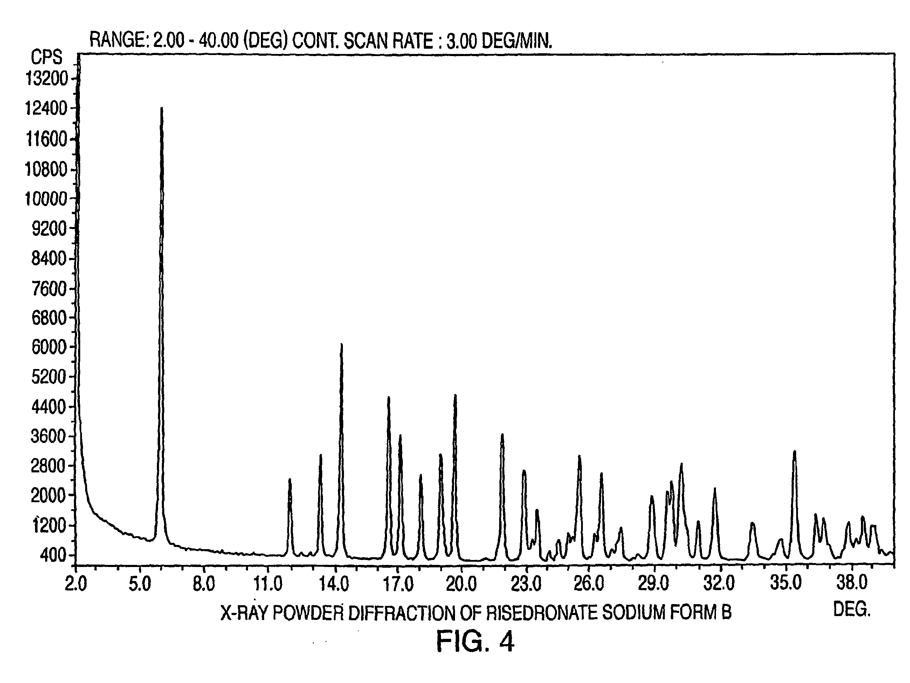

One characteristic of form B is its x-ray diffraction pattern. Form B is characterized by x-ray diffraction peaks at 2θ values of about 6.0, 14.4, 19.6, 24.9, and 25.4 degrees. The x-ray diffraction diagram for form B is shown in Figure 4. Principle x-ray diffraction peaks (reflections) for risedronate sodium form B are collect with those of other crystal forms in Table I.

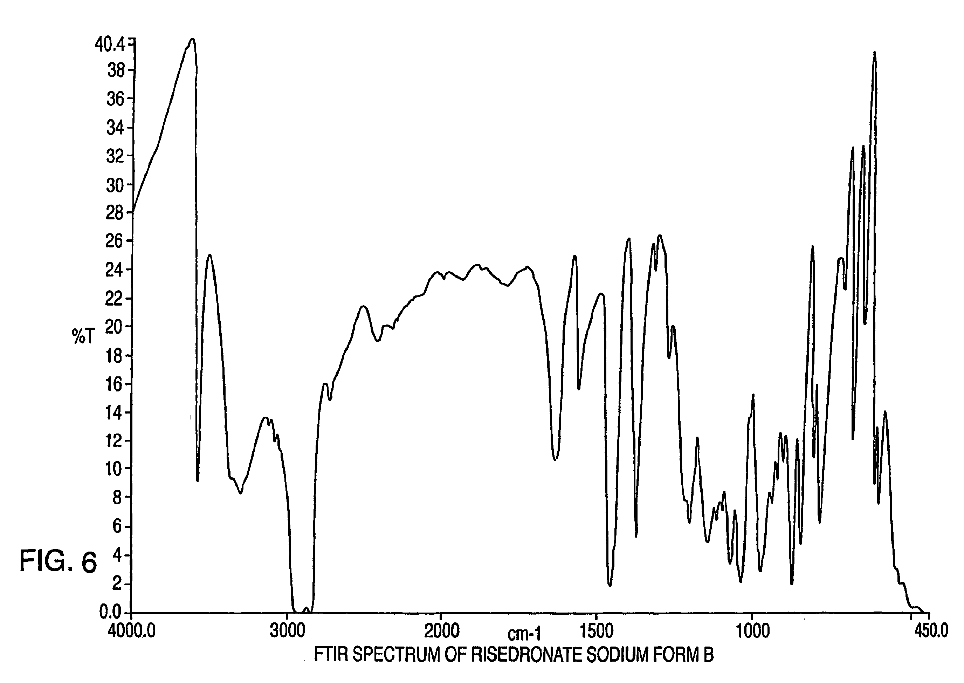

Another characteristic of form B is its absorption bands in FTIR. The FTIR spectrum of form B is shown in figure 6. Absorption bands characteristic of form B include those at 624, 951, 796, 912, 931, 1046, 1105, 1123, 1323, and 1641 cm⁻¹. FTIR absorption bands characteristic for form B are collected with those of other crystalline forms of risedronate sodium in Table III.

In another embodiment the present invention provides pure risedronate sodium form B. Pure risedronate sodium form B of the present invention is also physically stable and does not substantially convert (transform) to form A hemipentahydrate when exposed to 75% to 100% RH for one week or more; or upon storage at 40°C and 75%RH for 6 months. Pure risedronate sodium has less than about 1% by weight form A.

In another embodiment, the present invention provides risedronate sodium form B that is stable against transformation to form A.

In another embodiment, the present invention provides risedronate sodium having the characteristics of form BB. Form BB has a TGA weight loss between about 9% and about 11%.

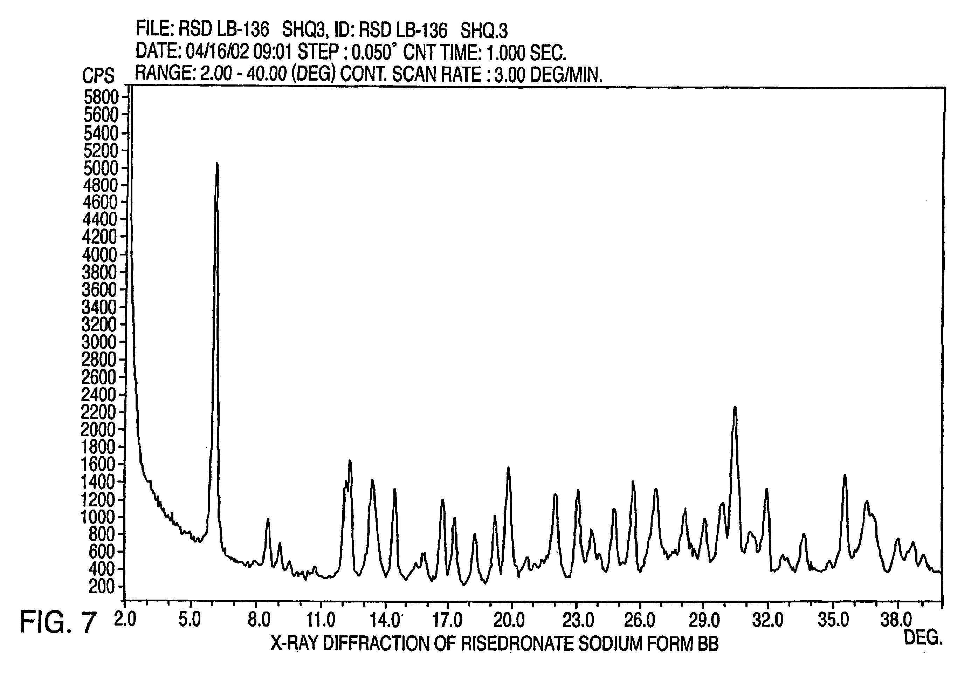

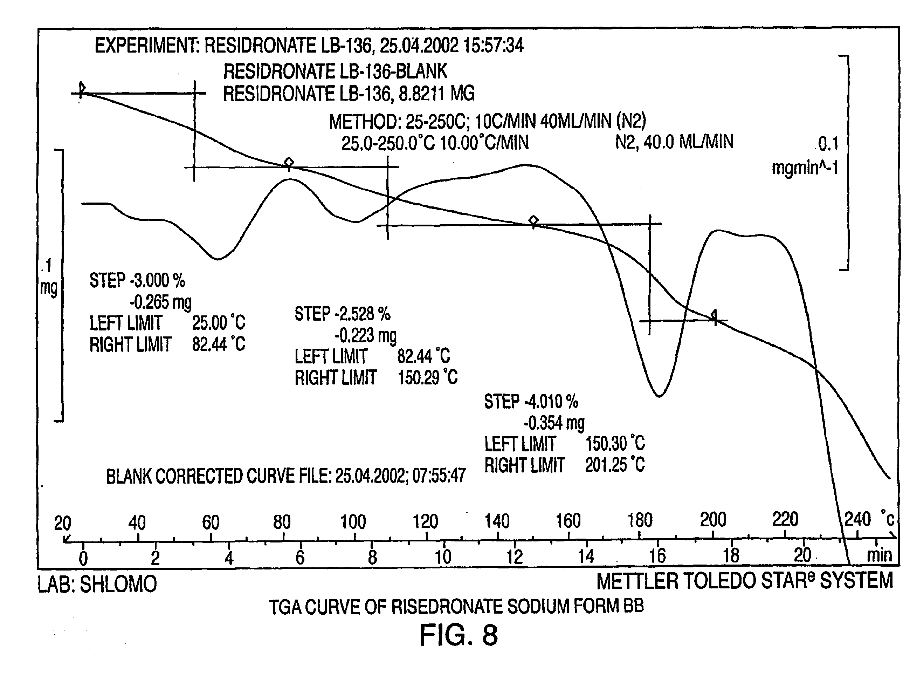

One characteristic of form BB is its x-ray diffraction pattern. Form BB has characteristic x-ray diffraction peaks at 2θ values of about 8.5, 9.1, 9.5, and 12.2 degrees θ and other peaks at 14.3, 16.9, 19.7, 23.5, 28.8, and 33.6 degrees 2θ. The x-ray diffraction diagram of form BB is shown in Figure 7. The TGA curve for risedronate sodium form BB is shown in Figure 8.

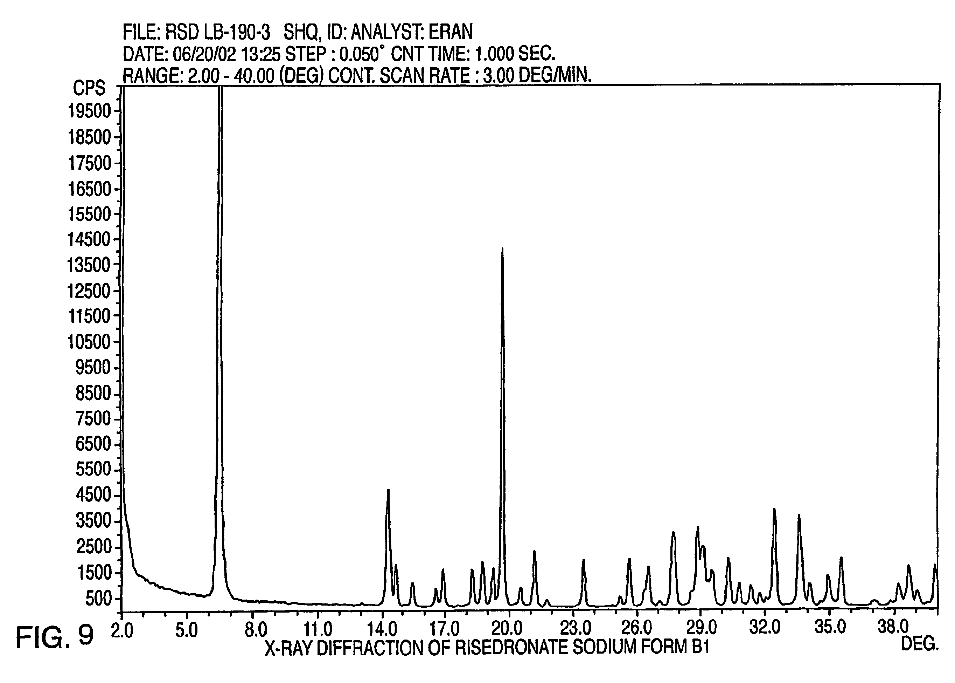

In another embodiment, the present invention provides risedronate sodium having characteristics of form B1. One characteristic of form B1 is its x-ray diffraction diagram. Form B1 is characterized by x-ray diffraction peaks at 2θ values at about 6.5, 14.7, 21.2, 27.7, and 32.4 degrees 2θ and is further characterized by x-ray diffraction peaks at 14.3, 16.9, 19.7, 23.5, 28.8, and 33.6 degrees 2θ. The x-ray diffraction diagram of form B1 is shown in Figure 9. The characteristic x-ray diffraction peaks for form B1 are collected with those for other risedronate sodium crystal forms in Table II. Form B1 is a disodium salt.

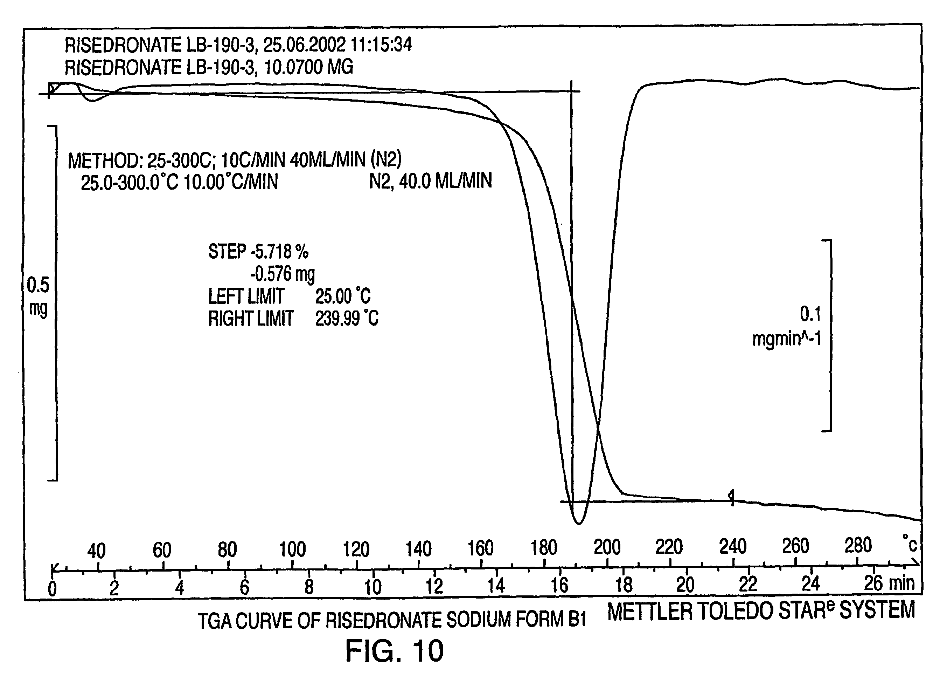

The TGA thermogram for form B1 is shown in Figure 10.

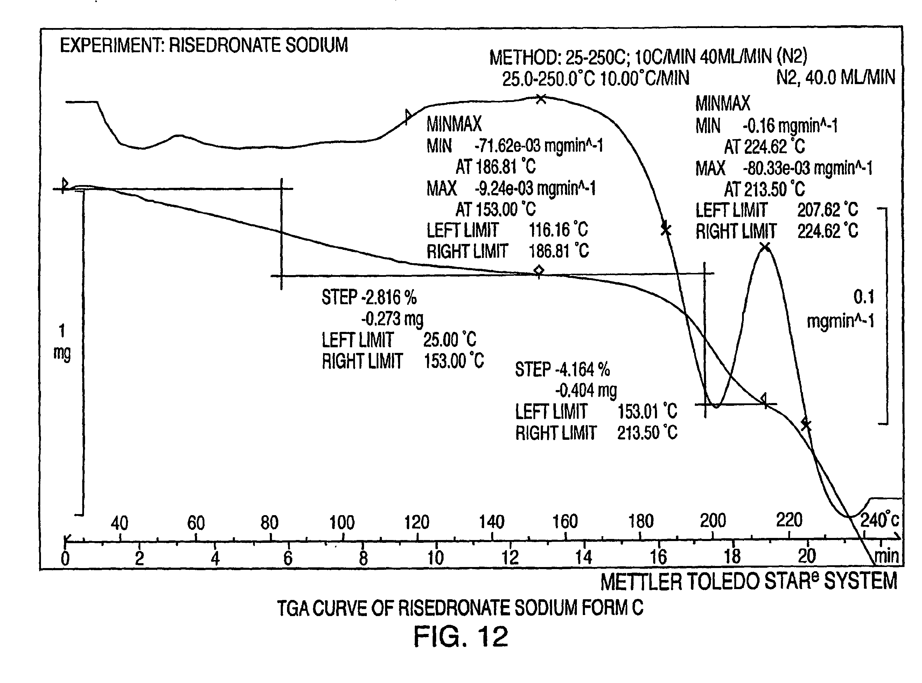

In another embodiment, the present invention provides risedronate sodium having the characteristics of form C. Form C has a TGA weight loss of about 7%. The TGA curve for form C is shown in Figure 12. Form C is characterized by x-ray diffraction peaks at 20 values of about 5.6, 10.3, 12.9, 16.5, and 30.9. The locations of characteristic x-ray peaks for form C are collected with those of other crystal forms of risedronate sodium in Table II.

Another characteristic of form C is its absorption bands in FTIR spectroscopy. Risedronate sodium form C is characterized through its FTIR spectrum. Characteristic absorption band for form C include those at 615, 666, 1089, 1563, and 1625 cm⁻¹. FTIR absorption bands characteristic of form C are collected with those of other crystalline forms of risedronate sodium in Table III.

Form C does not substantially convert (transform) to form A in high humidity atmosphere of 80%-100% at room temperature, preferably 80% relative humidity humidities for at least a period of one week.

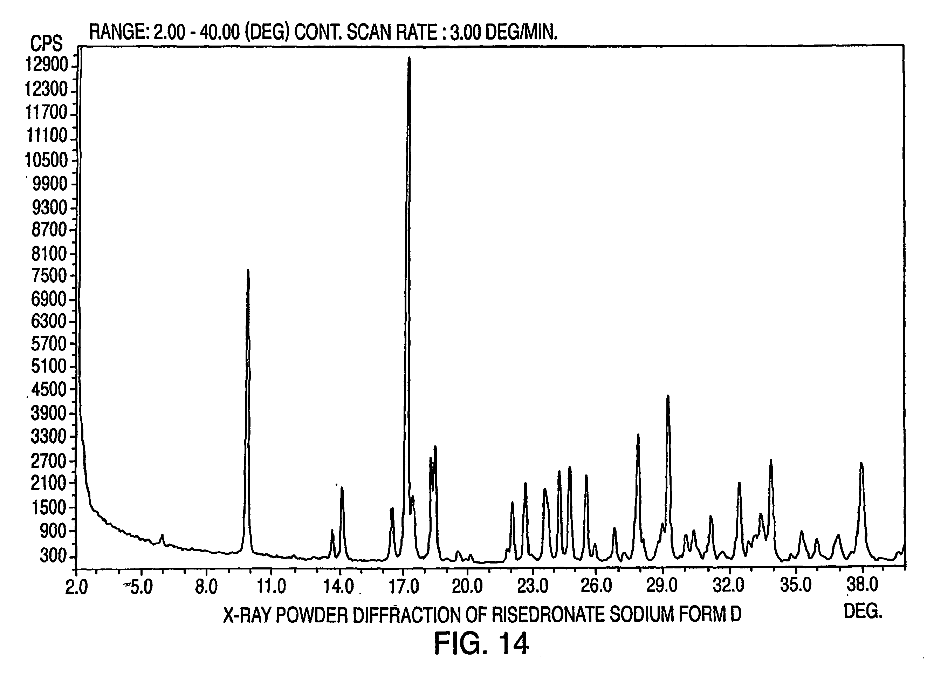

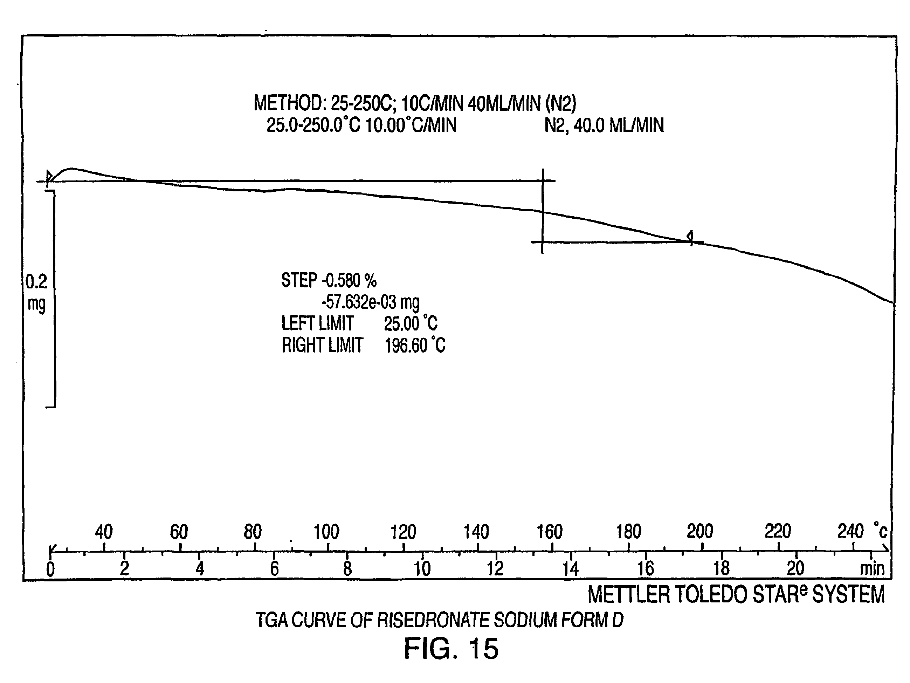

In another embodiment, the present invention provides risedronate sodium having the characteristics of form D, which has a TGA weight loss of not more than about 3%. The TGA curve for form D is shown in Figure 15. One characteristic of form D is its x-ray diffraction pattern. Form D is characterized by x-ray diffraction peaks at 2θ values of about 9.9, 17.2, 22.1, 27.9, and 29.2 degrees. The x-ray diffraction diagram of form D is shown in Figure 14. The locations of characteristic x-ray peaks for form D are collected with those of other polymorphs of risedronate sodium in Table II.

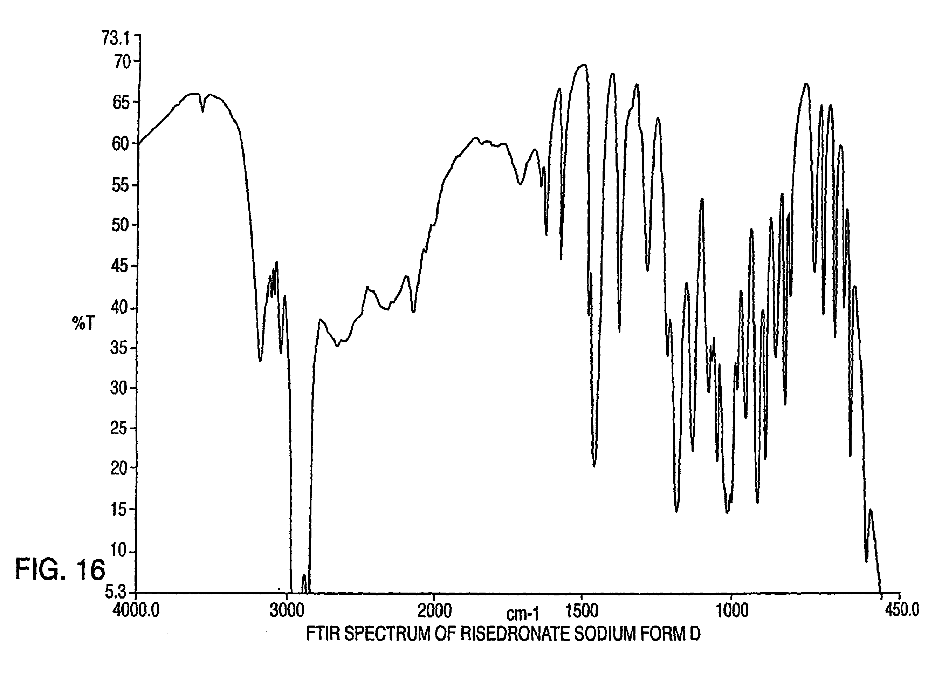

Another characteristic of form D is its absorption bands in FTIR. Risedronate sodium form D is characterized by means of its FTIR spectrum. The FTIR spectrum for form D is shown in Figure 16. Absorption bands characteristic of form D include those at 697, 807, 854, 955, 1187, 1218, 1576, 1646, and 1719 cm⁻¹. FTIR absorption bands for form D are collected with those of other crystalline forms of risedronate sodium in Table III.

Form D does not substantially convert (transform) to form A in high humidity atmosphere of 80%-100% RH at room temperature for at least a period of one week. Form D can be converted to form B by exposing it to an atmosphere of 80% to 100% RH for at least about one week.

In yet another embodiment, the present invention provides risedronate sodium having the characteristics of form E, which can be characterized by x-ray diffraction peaks at 2θ values of about 8.4, 8.9, 13.6, 27.6, and 27.9 degrees and a TGA weight loss of 9% to 12%. The locations of the characteristic diffraction peaks for form E are collected with those of other crystalline forms of risedronate sodium in Table II.

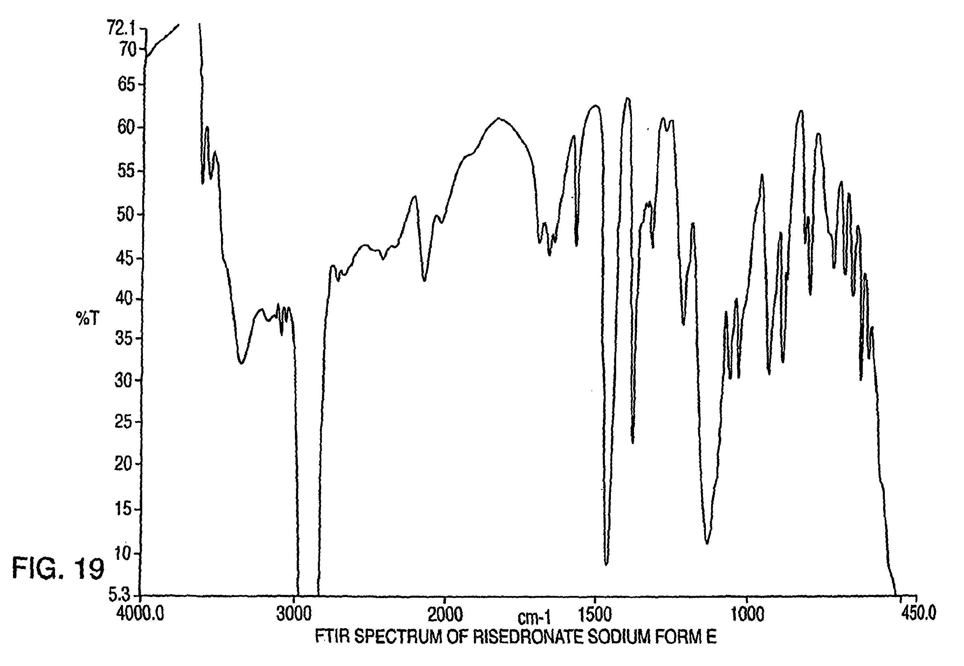

Another characteristic of form E is its absorption bands in FTIR spectroscopy. The FTIR spectrum for form E is shown in Figure 19. Absorption bands characteristic of form E include those at 801, 890, 935, 1656, and 1689 cm⁻¹. Characteristic FTIR absorption bands for form E are collected with those of other crystalline forms of risedronate sodium in Table III.

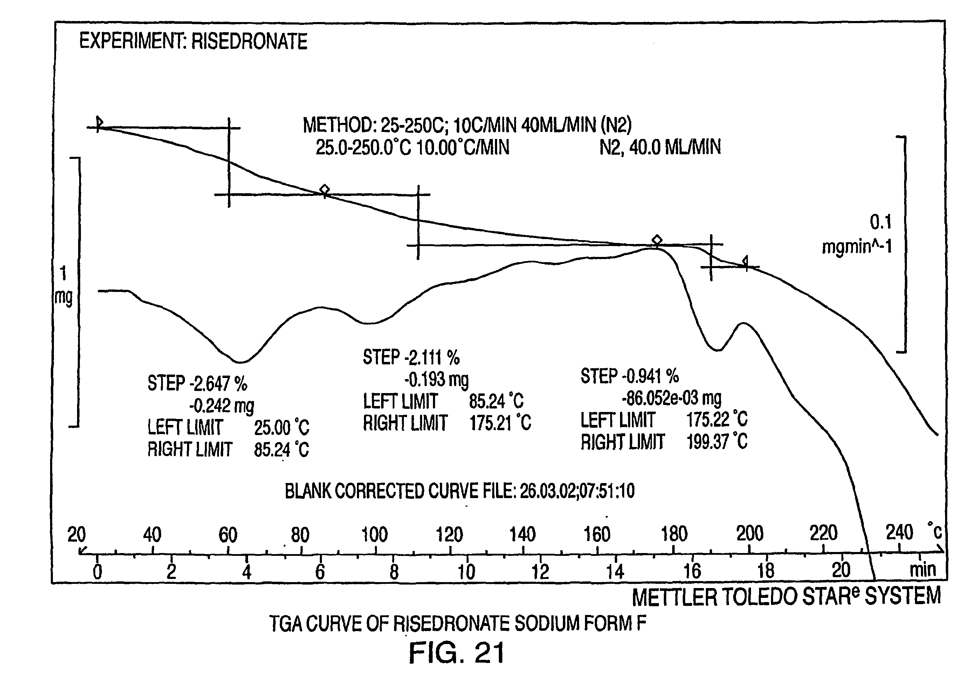

In another embodiment, the present invention provides risedronate sodium having the characteristics of form F, which has a TGA weight loss between about 4% and about 6%. The TGA curve for form F is shown in figure 21.

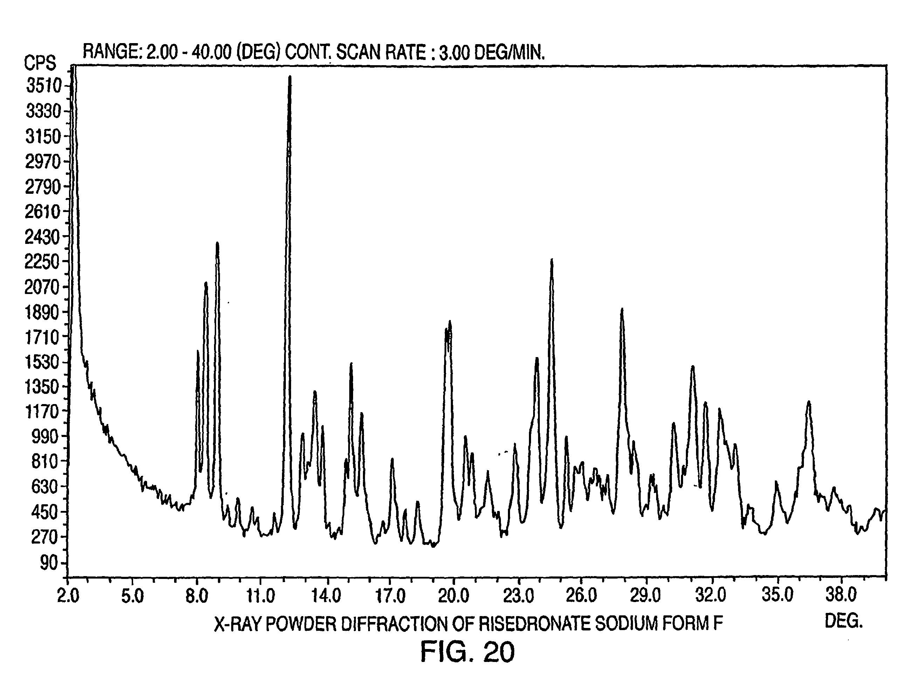

One characteristic of form F is its x-ray diffraction pattern. Risedronate sodium form F is characterized by x-ray diffraction peaks at values of 2θ of about 6.6, 8.4, 8.9, 12.2, and 18.6 degrees. The x-ray diffraction diagram of form F is shown in Figure 20. The locations of characteristic x-ray peaks for form F are collected with those of other crystalline forms of risedronate sodium in Table II.

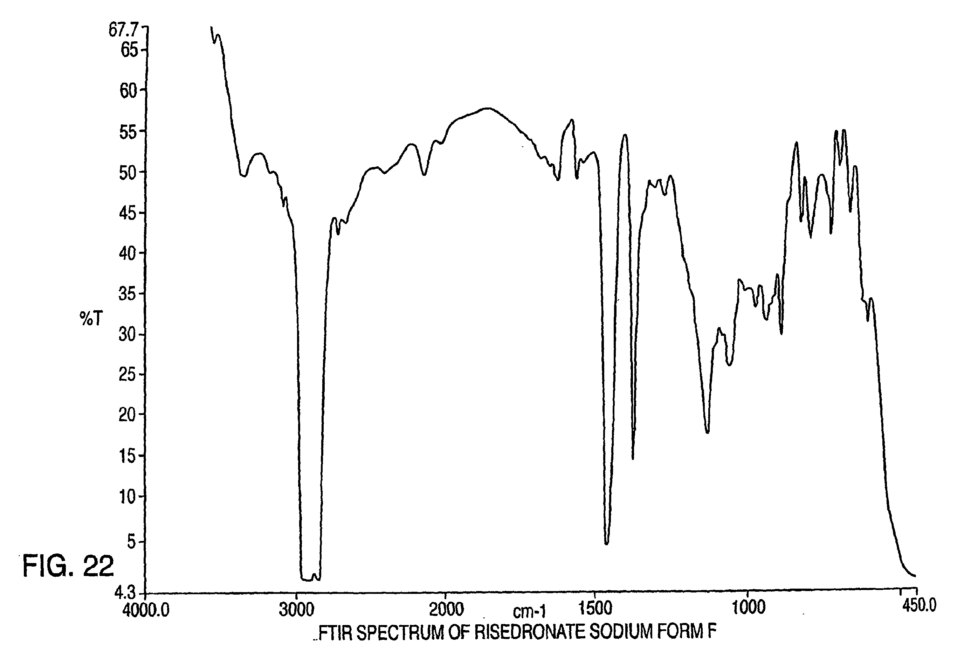

Risedronate sodium form F can be further characterized by means of FTIR spectroscopy. The FTIR spectrum of form F is shown in figure 22. Absorption bands characteristic of form F include those 971, 1133, and 1306 cm⁻¹. Characteristic absorption bands of FTIR absorptions for form F are collected with those of other crystalline forms of risedronate sodium in Table III.

Form F does not substantially rearrange (transform) to the hemipentahydrate form at up to 100% relative humidity during one week storage. In high humidity atmosphere of 80%-100% RH form F transforms to form BB during storage of one week or more.

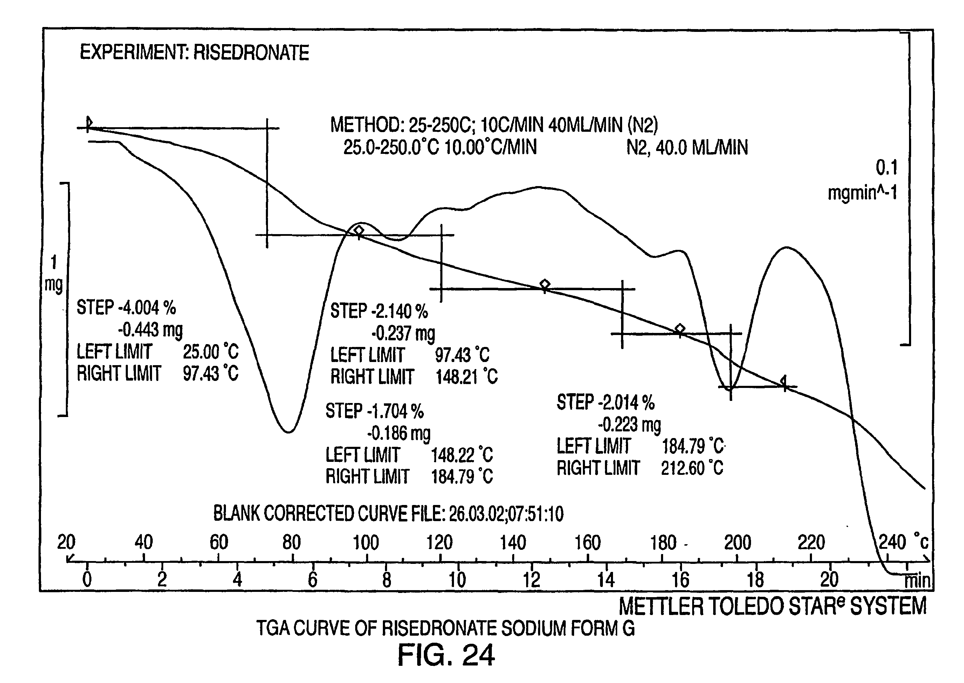

In yet another embodiment, the present invention provides risedronate sodium having the characteristics of form G, which has a TGA weight loss between about 9% and about 11%. The TGA curve of form G is shown in Figure 24.

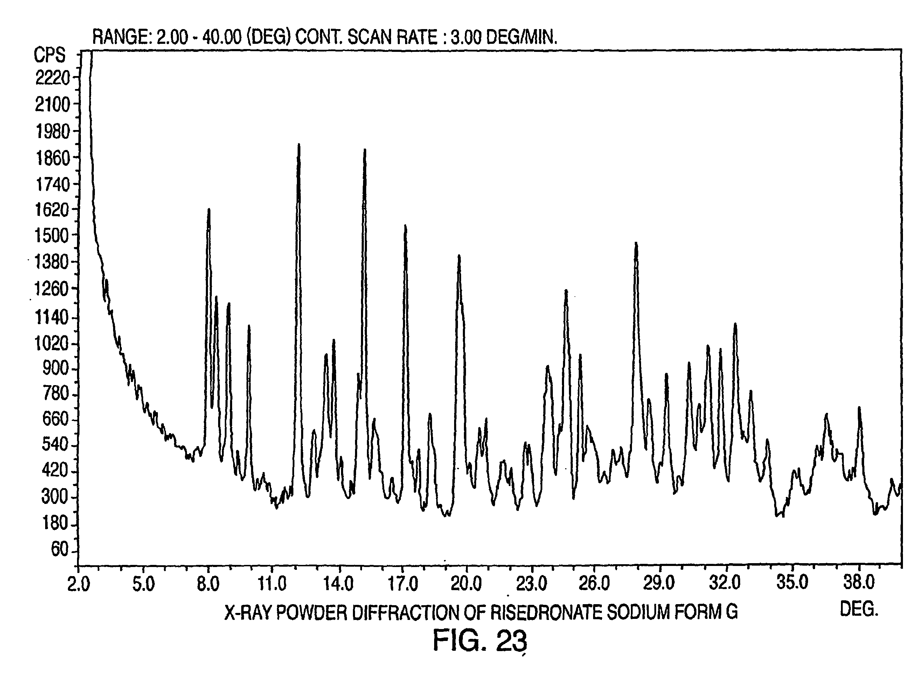

One characteristic of form G is its x-ray diffraction pattern. Risedronate sodium form G is characterized by x-ray diffraction peaks at 2θ values of about 8.0, 9.9, 12.2, 15.2, and 19.6 degrees. The x-ray diffraction diagram of form G is shown in figure 23. The locations of the characteristic diffraction peaks of form G are collected with those of other crystalline forms of risedronate sodium in Table II.

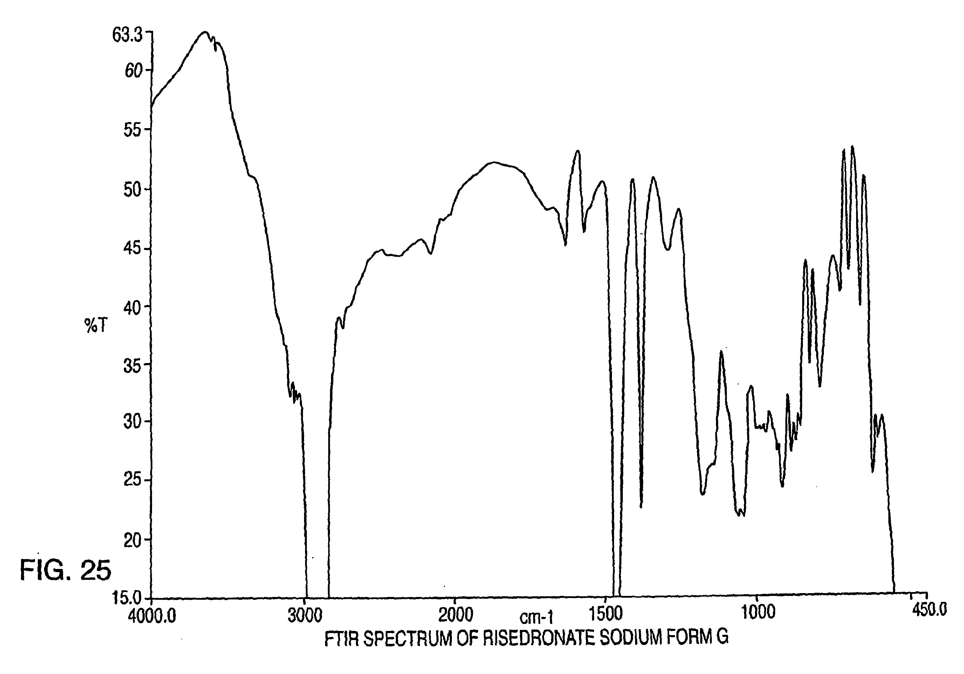

Another characteristic of form G is its absorption bands in FTIR spectroscopy. Risedronate sodium form G can be further characterized by FTIR spectroscopy. The FTIR spectrum of form G is shown in Figure 25. Characteristic FTIR absorption bands of form G include those at 724, 871, 1174,and 1285 cm⁻¹. Characteristic FTIR absorption bands for form G are collected with those for other crystalline forms of risedronate sodium in Table III.

In yet another embodiment, the present invention provides risedronate sodium having the characteristics of form H, which can be characterized by x-ray diffraction peaks at values of 2θ of about 6.9, 9.8, 10.9, 13.7, 16.0, and 18.0 degrees. The characteristic x-ray diffraction peaks for form H are collected with those of other crystalline forms of risedronate sodium in Table II.

Form H is further characterized by a TGA weight loss of 22%, which corresponds to the water content of pentahydrate.

**Table II**

| Risedronate sodium XRD peaks (degrees 2-theta) | | | | | | | | | |
|---|---|---|---|---|---|---|---|---|---|
| Form A (hemipenta) | Form B | Form B1 | Form BB | Form C | Form D | Form E | Form F | Form G | Form H |
| 8.9 | 6.0 | 6.5 | 5.9 | 5.6 | 9.9 | 6.5 | 8.0 | 8.0 | 6.9 |
| 12.2 | 12.0 | 14.3 | 8.5 | 10.3 | 13.8 | 7.4 | 8.4 | 8.3 | 9.8 |
| 12.9 | 13.4 | 14.7 | 9.1 | 12.9 | 14.2 | 8.4 | 8.9 | 8.9 | 10.9 |
| 13.5 | 14.4 | 16.9 | 9.5 | 15.2 | 16.5 | 8.9 | 12.2 | 9.9 | 13.7 |
| 15.4 | 16.5 | 19.7 | 12.2 | 16.5 | 17.2 | 13.6 | 14.0 | 12.2 | 16.0 |
| 15.7 | 17.1 | 21.2 | 16.7 | 17.6 | 18.3 | 14.4 | 15.6 | 13.5 | 17.2 |
| 19.8 | 18.1 | 23.5 | 22.0 | 17.8 | 18.5 | 16.4 | 16.9 | 13.8 | 18.0 |
| 22.9 | 19.0 | 27.7 | 24.7 | 20.3 | 22.1 | 20.1 | 19.8 | 15.2 | 19.0 |
| 24.6 | 19.6 | 28.8 | 28.0 | 20.9 | 22.7 | 22.9 | 18.6 | 17.1 | 19.5 |
| 27.8 | 21.8 | 32.4 | 20.5 | 21.2 | 23.6 | 23.9 | 21.5 | 19.6 | 20.9 |
| 28.1 | 24.9 | 33.6 | 23.9 | 26.0 | 24.3 | 24.6 | 27.8 | 23.7 | 23.2 |
| 31.3 | 25.4 | | 24.6 | 26.7 | 24.7 | 27.6 | | 24.6 | 25.2 |
| 36.5 | 26.5 | | 27.8 | 28.8 | 27.9 | 27.9 | | 25.2 | 25.9 |
| | 30.2 | | 30.2 | 29.6 | 29.2 | 28.5 | | 27.8 | 26.4 |
| | | | | 30.9 | 33.9 | 30.2 | | 32.4 | 27.5 |
| | | | | | | | | | 30.0 |

**Table III**

| Risedronate FTIR Peaks (in cm⁻¹) | | | | | | |
|---|---|---|---|---|---|---|
| Pentahydrate (form A) | Form B | Form C | Form D | Form E | Form F | Form G |
| 604 | 610 | 615 | 608 | 604 | 604 | 607 |
| 629 | 624 | 666 | 630 | 630 | 661 | 623 |
| 660 | 660 | 815 | 660 | 660 | 694 | 659 |
| 687 | 692 | 888 | 697 | 687 | 791 | 694 |
| 800 | 951 | 972 | 807 | 801 | 824 | 724 |
| | 796 | 1016 | 825 | 819 | 888 | 792 |
| 818 | 817 | 1052 | 854 | 890 | 937 | 822 |
| 889 | 858 | 1089 | 889 | 934 | 971 | 859 |
| | 884 | 1151 | 915 | 1033 | 1061 | 871 |
| 915 | 912 | 1273 | 955 | 1212 | 1133 | 886 |
| 935 | 931 | 1563 | 985 | 1275 | 1275 | 916 |
| 1023 | 945 | 1615 | 1017 | 1212 | 1306 | 934 |
| 1032 | 983 | 1682 | 1052 | 1275 | 1568 | 945 |
| 1061 | 1046 | | 1082 | 1318 | 1628 | 970 |
| 1132 | 1080 | | 1136 | 1569 | | 985 |
| 1212 | 1105 | | 1187 | 1638 | | 1043 |
| 1275 | 1123 | | 1218 | 1656 | | 1060 |
| 1319 | 1152 | | 1283 | 1689 | | 1174 |
| 1568 | 1210 | | 1576 | | | 1285 |
| 1637 | 1276 | | 1627 | | | 1567 |
| 1657 | 1323 | | 1646 | | | 1627 |
| 1689 | 1567 | | 1719 | | | |
| | 1641 | | | | | |

The novel crystal forms (polymorphs and pseudopolymorphs) of the present invention can be prepared by several methods. These methods include a reflux method, an annealing (thermal) method, and a humidification method.

The reflux method is preferred when risedronate sodium having the characteristics of any of forms B, BB, B1, C, D, or E is desired. In the reflux method, risedronic acid is combined with a suitable base having sodium as a counterion (cation) and a reflux medium that can be water, an alcohol, or a mixture of an alcohol and water. Sodium hydroxide is a preferred sodium base. The particular alcohol and the composition of the alcohol - water mixture are chosen according to the form desired. The following table (Table III) will provide guidance to the skilled artisan in selecting the appropriate reflux medium. The composition of reflux media are expressed on a volume / volume basis (abbreviated v/v). That is, 50% v/v means a mixture of approximately equal volumes.

**Table III**

| Polymorph Produced in the Reflux Method Using Various Reflux Media (Alcohols and Alcohol - Water Mixtures) | | | |
|---|---|---|---|
| % H₂O (v/v) | MeOH | EtOH | IPA |
| 0 | D | D | Risedronic acid |
| 3 | | C | |
| 10 | D | B >> B1 | B+risedronic acid |
| 20 | B | B >> B1 | B+risedronic acid |
| 40 | B | B | B |
| 60 | B | B | B |
| 70 | B | B+A | |
| 80 | E | A+E | A |
| 100 | A+E | | |

Generally, 1 to 2 equivalents of sodium hydroxide are combined with risedronic acid and water, alcohol, or an alcohol- water mixture. The total volume of reflux medium is not critical and can be, for example, between about 15 and about 25 milliliters per gram of risedronic acid used. In preferred embodiments, a solution of sodium hydroxide in the chosen reflux medium is added to a suspension of risedronic acid in the reflux medium (12 to 22 mL per gram of risedronic acid are convenient). The mixture is refluxed for 0.5 to 30 hours, preferably 3 to 24 hours. Optionally but preferably, the mixture is then cooled to room temperature and then to less than about 5°C, most preferably to about 0°C. The solids are separated and collected by any suitable means, such as filtration (gravity or suction) or centrifugation, to mention just two.

The annealing (incubating) method is preferred when forms F and G are desired. Annealing can be accomplished by exposing the starting risedronate sodium to the desired temperature for the desired time using, for example, an oven. The skilled artisan will know to adjust the annealing time according to the temperature chosen. Lower annealing temperatures generally require longer annealing times.

In the practice of the present invention, risedronate sodium having at least one characteristic of form B can be made by refluxing a combination of risedronic acid, a sodium base, and a liquid that is a mixture with water of an alcohol selected from methanol, ethanol, n-propanol, and iso-propanol.

In a particular embodiment, the present invention provides a process by which form B is obtained, by combining at reflux temperature a suspension of risedronic acid in a mixture of water and an alcohol, preferably ethanol, methanol, or isopropanol with a solution of sodium hydroxide in a mixture of alcohol and water. The reaction to yield product having the characteristics of form B takes place for a period of 0.5-30 hours, more preferably 20 hours.

In another embodiment, the present invention provides a process by which form B is obtained by combining, at reflux temperature, risedronic acid and about one to about one-and-one half equivalents of sodium hydroxide in mixture of water and ethanol (40% to 60%, especially 50%, v/v, water in ethanol, *see* Table III), wherein the combination is a suspension.

In another embodiment, the present invention provides a process by which risedronate sodium having at least one characteristic of form B is obtained, by combining at reflux temperature a suspension of risedronic acid in a mixture of water and methanol (20% to 70%, v/v, methanol, see Table III) with a solution of about one to about one-and-one half equivalents of sodium hydroxide in a mixture of alcohol and water.

In another embodiment, the present invention provides a process by which risedronate sodium having at least one characteristic of form B is obtained, by combining at reflux temperature a suspension of risedronic acid in a liquid that is a mixture of water and isopropanol (40% to 60%, v/v, isopropanol) with a solution of sodium hydroxide in a mixture of alcohol and water.

In yet another embodiment, the present invention provides a method of making risedronate sodium having at least one characteristic of form B including the step of treating risedronate sodium with a lower alkanol at a temperature between about room temperature and reflux temperature. Treatment can be effected by agitating a combination of risedronate sodium having at least one characteristic of form A and a lower alkanol for a time sufficient to effect the transformation. The skilled artisan will know to optimize the treatment time by routine experimentation by monitoring the transformation using, for example, x-ray diffraction analysis.

Another embodiment of the invention provides a process by which form B, alone or in admixture with form B 1 is obtained, by combining at reflux temperature risedronic acid and one equivalent sodium hydroxide in an ethanol water mixture (5% - 25%, v/v, water in ethanol) for a period of 5-20 hours, most preferably 10 hours.

In another embodiment, the present invention provides a method for making risedronate sodium having at least one characteristic of form B1 including the step of refluxing risedronic acid and two equivalents of an inorganic base, having sodium as the cation preferably NaOH in a 50/50 (v/v), alcohol/water, for 5 to 20, preferably 10 hours. With the appropriate reflux medium, use of at least about 2 equivalents of an inorganic base having sodium as the cation (e.g. NaOH) ensures that the product having the characteristics of B1 is formed in preference to form B.

Another aspect of the invention is the process by which risedronate sodium having at least one characteristic of form C is obtained, by combining at reflux temperature risedronic acid and sodium hydroxide in an ethanol-water mixture (3%water in ethanol, v/v) for a period of 10-30 hours, most preferably about 20 hours.

In another embodiment, the present invention provides a process by which risedronate sodium having at least one characteristic of form D is obtained by combining, at reflux temperature, risedronic acid and sodium hydroxide in methanol or ethanol or in a mixture of methanol and water (up to 11 %water v/v) for a period of 10-30 hours, most preferably about 20 hours.

In yet another embodiment the present invention provides a method by which form E, alone or in admixture with form A, is obtained by combining at reflux temperature risedronic acid and sodium hydroxide in a liquid that is water or a mixture of alcohols, preferably ethanol or methanol more preferably ethanol in water (up to 20% v/v ethanol) or methanol in water (up to 20% v/v methanol). The reaction to yield a product exhibiting the characteristics of form E takes place for a period of at least 1 hour.

In another embodiment, the present invention provides a method by which risedronate sodium having at least one characteristic of form BB is obtained by providing a solution of risedronate sodium in water at elevated temperature, preferably at 70°C, and adding IPA to the solution to obtain a suspension, isolating the suspended solid, drying the isolated precipitate, and suspending the dried precipitate in IPA at reflux for at least about 10 hours, especially about 17 hours.

Another aspect of the invention is the process by which risedronate sodium having at least one characteristic of form F is obtained, by heating a mixture of forms B and A at temperatures in the range 100-200°C, most preferably 120-180°C, most preferably 160°C. The time needed for the conversion depends on the temperature. At 160°C a period of 2-10 hours is used, preferably 5-8 hours.

Another embodiment of the invention is the process by which risedronate sodium having at least one characteristic of form G is obtained, by heating a mixture of forms A and E at temperatures in the range 100-200°C, most preferably 120-180°C, most preferably 160°C. The time needed for the conversion depends on the temperature. At 160°C a period of 2-10 hours is used, preferably 5-8 hours.

Another embodiment of the invention is the process by which risedronate sodium having at least one characteristic of form H is obtained, by exposing at high relative humidity, in the range 60-100% relative humidity, preferably 80% relative humidity, risedronate sodium form C. The period needed for the conversion is 3-20 days, preferably 7-14 days.

In another embodiment, the present invention provides a method for preparation of risedronate sodium having at least one characteristic of form A by combining at reflux temperature risedronic acid and sodium hydroxide in aqueous solution of isopropanol(80-100% v/v water). Under these conditions, formation of product exhibiting the characteristics of form A is usually complete in about 2 hours or less.

In another embodiment, the present invention provides a method for the preparation of form BB by exposing form F to high relative humidity of 60-100% RH, more preferrably 80% RH, for a period of time between 3 and 10 days, more preferrably 7 days.

In still another embodiment, the present invention provides a method for the preparation of risedronate sodium having at least one characteristic of form A by exposing form G to high relative humidity of 60-100% RH, more preferrably 80% RH, for a period of time between 3 and 10 days, more preferrably 7 days.

In yet another embodiment, the present invention provides a method for the preparation of risedronate sodium having at least one characteristic of form A by exposing to high relative humidity of 60-100% RH, more preferrably 80% RH, risedronate sodium having at least one characteristic of either form E or form G for a period of time between 3 and 10 days, more preferably 7 days.

Another embodiment of the invention is preparation of risedronate sodium having at least one characteristic of form B by exposing risedronate sodium having at least one characteristic of form D to high relative humidity of 60-100% RH, more preferrably 80% RH, for a period of time between 3 and 20 days, more preferably 5-10 days.

In another embodiment, the present method provides a method for the preparation of risedronate sodium having at least one characteristic of form H by exposing form C to high relative humidity of 60-100% RH, more preferrably 80% RH, for a period of time between 3 and 20 days, more preferably 5-10 days.

The humidification method is preferred when form H is desired.

In still a further embodiment, the present invention provides a method for the preparation of a risedronate sodium having at least one characteristic of form A by incubating form E at temperatures in the range 30-100°C, most preferably 50-80°C, most preferably 60°C. The time needed for the conversion depends on the temperature. At 60°C a period of 10-30 hours is used, preferably 20 hours.

In another embodiment, the present invention provides a method of making risedronate sodium having at least one characteristic of form A including the steps of treating risedronate sodium form B or form D with water at a temperature from room temperature up to about reflux temperature. Treatment can be by agitation of a mixture of form B or D and water as a slurry or suspension. The risedronate sodium is treated for a time sufficient to effect the transformation. The skilled artisan will know to optimize the treatment time by routine experimentation by monitoring the transformation by, for example, x-ray diffraction analysis.

In still another embodiment, the present invention provides a pharmaceutical composition including at least one ofrisedronate sodium forms B, B1, BB, C, D, E, F, G, or H and at least one pharmaceutically acceptable excipient. Preferably the pharmaceutical composition is in the form of an oral solid dosage form.

In yet another embodiment, the present invention provides a pharmaceutical composition including stable risedronate sodium form B and at least one pharmaceutically acceptable excipient. Preferably the pharmaceutical composition is in the form of an oral solid dosage form.

Tablets were prepared according to standard formulations and form B in the tablets was found to be stable for 5 months under stressed conditions of 40° C and 75% RH. The stability of risedronate sodium form B in tablets is not limited to particular formulations.

In yet another embodiment, the present invention provides a pharmaceutical composition including pure risedronate sodium form B and at least one pharmaceutically acceptable excipient. Preferably the pharmaceutical composition is in the form of an oral solid dosage form.

Risedronate sodium having at least one characteristic of any form herein described may be formulated into a variety of pharmaceutical compositions and dosage forms that are useful in treating patients afflicted with, for example, osteoporosis.

Pharmaceutical compositions of the present invention contain one or more of the herein described polymorphs of risedronate sodium. In addition to the active ingredient(s), risedronate sodium pharmaceutical compositions of the present invention may contain one or more excipients. Excipients are added to the composition for a variety of purposes.

Diluents increase the bulk of a solid pharmaceutical composition and may make a pharmaceutical dosage form containing the composition easier for the patient and caregiver to handle. Diluents for solid compositions include, for example, microcrystalline cellulose (e.g. AVICEL^{®}, microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. EUDRAGIT^{®}), potassium chloride, powdered cellulose, sodium chloride, sorbitol and talc.

Solid pharmaceutical compositions that are compacted into a dosage form like a tablet may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. KLUCEL®), hydroxypropyl methyl cellulose (e.g. METHOCEL®), liquid glucose, magnesium aluminum silicate, maltodextrin, methylcellulose, polymethacrylates, povidone (e.g. KOLLIDON®, PLASDONE®), pregelatinized starch, sodium alginate and starch. The dissolution rate of a compacted solid pharmaceutical composition in the patient's stomach may be increased by the addition of a disintegrant to the composition.

Disintegrants include alginic acid, carboxymethylcellulose calcium, carboxymethylcellulose sodium (e.g. AC-DI-SOL®, PRIMELLOSE®), colloidal silicon dioxide, croscarmellose sodium, crospovidone (e.g. KOLLIDON®, POLYPLASDONE®), guar gum, magnesium aluminum silicate, methyl cellulose, microcrystalline cellulose, polacrilin potassium, powdered cellulose, pregelatinized starch, sodium alginate, sodium starch glycolate (e.g. EXPLOTAB®) and starch.

Glidants can be added to improve the flow properties of non-compacted solid compositions and improve the accuracy of dosing. Excipients that may function as glidants include colloidal silicon dixoide, magnesium trisilicate, powdered cellulose, starch, talc and tribasic calcium phosphate.

When a dosage form such as a tablet is made by compaction of a powdered composition, the composition is subjected to pressure from a punch and die. Some excipients and active ingredients have a tendency to adhere to the surfaces of the punch and die, which can cause the product to have pitting and other surface irregularities. A lubricant can be added to the composition to reduce adhesion and ease release of the product from the die. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid ethyl maltol, and tartaric acid.

Compositions may also be colored using any pharmaceutically acceptable colorant to improve their appearance and/or facilitate patient identification of the product and unit dosage level.

Selection of excipients and the amounts to use may be readily determined by the formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and lozenges as well as liquid syrups, suspensions and elixirs. An especially preferred dosage form of the present invention is a tablet.

The present invention can be further demonstrated with the following non-limiting examples.

In the following examples, the identity of the particular polymorph formed was determined by x-ray diffraction and, where appropriate, TGA and FTIR.

### Preparation of crystal form B in aqueous Ethanol

### Example 1

A solution of sodium hydroxide (1.18g, 1 eq.) in a mixture of water/ethanol (32% v/v) (18.5ml) was added dropwise to a suspension of risedronic acid (8.35g) in a mixture of water/ethanol (32% v/v) (105ml) at reflux temperature. The reaction mixture was heated at reflux temperature for 18 hours. The reaction mixture was cooled to room temperature. Further cooling was performed using an ice-bath. The precipitate was then filtered, washed with ethanol (2x10ml) and dried in a vacuum oven at 50°C for 24 hours to give 8.77g (91%) of sodium risedronate crystal form **B.**

### Example 2

A solution of sodium hydroxide (1.47g, 1eq.) in water (100ml) was added in one portion to a suspension of Risedronic acid (10.0g) in Ethanol (100ml) at reflux temperature. The reaction mixture was heated at reflux temperature for additional 18 hours. Then the reaction mixture was cooled to room temperature. Further cooling was performed using an ice-bath. The precipitate was then filtered, washed with Ethanol (1x15ml) and dried in a vacuum oven at 50°C for 27 hours to give 9.35g of Sodium Risedronate crystal form B.

### Example 3

A solution of sodium hydroxide (1.18g, 1 eq.) in a mixture of water/Ethanol (60% v/v) (126ml) was added in one portion to dry solid risedronic acid (8.35g) at room temperature. The reaction mixture was then heated to reflux temperature for 18 hours. Then the reaction mixture was cooled to room temperature. Further cooling was performed using an ice-bath. The precipitate was then filtered, washed with Ethanol (1 x 10ml) and dried in a vacuum oven at 50°C for 24 hours to give 8.04g (84%) of Sodium Risedronate crystal form B (LOD by TGA=6.22%).

### Example 4

A solution of sodium hydroxide (1.38g, 1 eq.) in a mixture of water/Bthanol (50% v/v) (30ml) was added dropwise to a suspension of Risedronic acid (10.0g) in a mixture of water/Ethanol (50% v/v) (170ml) at reflux temperature. The reaction mixture was heated at reflux temperature until the pH was 4.10-4.30 (about 1 hour). The reaction mixture was cooled to room temperature. Further cooling was performed using an ice-bath. The precipitate was then collected by filtration, washed with a mixture of water/Ethanol (1:1) (1x20ml), and dried in a vacuum oven at 50°C for 14 hours to give 7.50g of Sodium Risedronate crystal form B.

### Example 5

A solution of sodium hydroxide (1.18g, 1 eq.) in a mixture of water/ethanol (39% v/v) (20.5ml) was added dropwise to a suspension of risedronic acid (8.35g) in a mixture of water/ethanol (39% v/v) (116ml) at reflux temperature. The reaction mixture was heated at reflux temperature for 18 hours. The reaction mixture was cooled to room temperature. Further cooling was performed using an ice-bath. The precipitate was then filtered, washed with ethanol (2x10ml) and dried in a vacuum oven at 50°C for 24 hours to give 8.50g (88%) of sodium risedronate crystal form **B.**

### Example 6

A solution of sodium hydroxide (1.18g, 1eq.) in a mixture of water/ethanol (60% v/v) (31ml) was added dropwise to a suspension of risedronic acid (8.35g) in a mixture of water/ethanol (60% v/v) (178ml) at reflux temperature. The reaction mixture was heated at reflux temperature for 19 hours. The reaction mixture was cooled to room temperature. Further cooling was performed using an ice-bath. The precipitate was then filtered, washed with ethanol (1 x20ml) and dried in a vacuum oven at 50°C for 20 hours to give 7.55g of sodium risedronate crystal form **B.**

### Preparation of crystal form B in aqueous Methanol

### Example 7

A solution of sodium hydroxide (1.18g,1eq.) in a mixture of water/methanol (24% v/v) (16.6ml) was added dropwise to a suspension of risedronic acid (8.35g) in a mixture of water/methanol (24% v/v) (94ml) at reflux temperature. The reaction mixture was heated at reflux temperature for 18.5 hours. The reaction mixture was cooled to room temperature. Further cooling was performed using an ice-bath. The precipitate was then filtered, washed with methanol (2x10ml) and dried in a vacuum oven at 50°C for 19 hours to give 8.69g of sodium risedronate crystal form **B.**

### Example 8

A solution of sodium hydroxide (1.18g,1eq.) in a mixture of water/methanol (39% v/v) (20.6ml) was added dropwise to a suspension of risedronic acid (8.35g) in a mixture of water/methanol (39% v/v) (117ml) at reflux temperature. The reaction mixture was heated at reflux temperature for 18.5 hours. The reaction mixture was cooled to room temperature. Further cooling was performed using an ice-bath. The precipitate was then filtered, washed with methanol (2x10ml) and dried in a vacuum oven at 50°C for 19 hours to give 8.30g of sodium risedronate crystal form **B.**

### Example 9

A solution of sodium hydroxide (1.18g,1eq.) in a mixture of water/methanol (60% v/v) (31.3ml) was added dropwise to a suspension of risedronic acid (8.35g) in a mixture of water/methanol (60% v/v) (178ml) at reflux temperature. The reaction mixture was heated at reflux temperature for 18 hours. The reaction mixture was cooled to room temperature. Further cooling was performed using an ice-bath. The precipitate was then filtered, washed with methanol (2x10ml) and dried in a vacuum oven at 50°C for 22 hours to give 7.75g of sodium risedronate crystal form **B**.

### Example 10

A solution of sodium hydroxide (1.18g, 1eq.) in a mixture of water/methanol (70% v/v) (41.8ml) was added dropwise to a suspension of risedronic acid (8.35g) in a mixture of water/methanol (70% v/v) (237ml) at reflux temperature. The reaction mixture was heated at reflux temperature for 19 hours. The reaction mixture was cooled to room temperature. Further cooling was performed using an ice-bath. The precipitate was then filtered, washed with methanol (2x10ml) and dried in a vacuum oven at 50°C for 23 hours to give 6.55g of sodium risedronate crystal form **B**.

### Preparation of crystal form B in aqueous 2-Propanol (IPA)

### Example 11

A solution of sodium hydroxide (1.18g, 1eq.) in a mixture of water/IPA (40% v/v) (21ml) was added dropwise to a suspension of risedronic acid (8.35g) in a mixture of water/IPA (40% v/v) (119ml) at reflux temperature. The reaction mixture was heated at reflux temperature for 19 hours. The reaction mixture was cooled to room temperature. Further cooling was performed using an ice-bath. The precipitate was then filtered, washed with IPA (1x25ml) and dried in a vacuum oven at 50°C for 27 hours to give 8.53g of sodium risedronate crystal form **B**.

### Example 12

A solution of sodium hydroxide (1.18g, 1eq.) in a mixture of water/IPA (60% v/v) (31 ml) was added dropwise to a suspension of risedronic acid (8.35g) in a mixture of water/IPA (60% v/v) (178ml) at reflux temperature. The reaction mixture was heated at reflux temperature for 19 hours. Then the reaction mixture was cooled to room temperature. Further cooling was performed using an ice-bath. The precipitate was then filtered, washed with IPA (1x25ml) and dried in a vacuum oven at 50°C for 23 hours to give 8.09g of sodium risedronate crystal form **B**.

### Preparation of crystal form C in aqueous Ethanol

### Example 13

A solution of sodium hydroxide (1.18g, 1eq.) in a mixture of water/ethanol (3% v/v) (12.4ml) was added dropwise to a suspension of risedronic acid (8.35g) in a mixture of water/ethanol (3% v/v) (70ml) at reflux temperature. The reaction mixture was heated at reflux temperature for 18 hours. The reaction mixture was cooled to room temperature. Further cooling was performed using an ice-bath. The precipitate was then filtered, washed with ethanol (2×10ml) and dried in a vacuum oven at 50°C for 22 hours to give 8.20g (89%)of sodium risedronate crystal form **C**.

### Preparation of crystal form D in aqueous Methanol

### Example 14

A solution of sodium hydroxide (1.18g, 1eq.) in a mixture of water/methanol (11% v/v) (14ml) was added dropwise to a suspension of risedronic acid (8.35g) in a mixture of water/methanol (11% v/v) (80ml) at reflux temperature. The reaction mixture was heated at reflux temperature for 18 hours. The reaction mixture was cooled to room temperature. Further cooling was performed using an ice-bath. The precipitate was then filtered, washed with methanol (1x10ml) and dried in a vacuum oven at 50°C for 20 hours to give 8.20g (90%)of sodium risedronate crystal form **D**.

### Preparation of crystal form E in aqueous Methanol

### Example 15

A solution of sodium hydroxide (1.18g, 1eq.) in a mixture of water/methanol (80% v/v) (62.6ml) was added dropwise to a suspension of risedronic acid (8.35g) in a mixture of water/methanol (80% v/v) (355ml) at reflux temperature. The reaction mixture was heated at reflux temperature for 2 hours. The reaction mixture was cooled to room temperature. Further cooling was performed using an ice-bath. The precipitate was then filtered, washed with methanol (1x10ml) and dried in a vacuum oven at 50°C for 24 hours to give 5.27g (51 %)of sodium risedronate crystal form **E**.

### Preparation of crystal form E in aqueous Ethanol

### Example 16

A solution of sodium hydroxide (1.18g, 1eq.) in a mixture of water/ethanol (80% v/v) (63ml) was added dropwise to a suspension of risedronic acid (8.35g) in a mixture of water/ethanol (80% v/v) (354.5ml) at reflux temperature. The reaction mixture was heated at reflux temperature for 19 hours. The reaction mixture was cooled to room temperature. Further cooling was performed using an ice-bath. The precipitate was then filtered, washed with ethanol (1x20ml) and dried in a vacuum oven at 50°C for 20 hours to give 6.17g of sodium risedronate crystal form **E** in a mixture with form **A.**

### Preparation of crystal form E in Water

### Example 17

A solution of sodium hydroxide (1.18g,1eq.) in water (12.5ml) was added dropwise to a suspension of risedronic acid (8.35g) water (71ml) at reflux temperature. The reaction mixture was heated at reflux temperature for 1 hour. The reaction mixture was cooled to room temperature and stirred at this temperature for 16 hours. Further cooling was performed using an ice-bath. The precipitate was then filtered, washed with ethanol (1x20ml) and dried in a vacuum oven at 50°C for 19 hours to give 6.01 g of sodium risedronate crystal form **E** in a mixture with crystal form **A.**

### Preparation of crystal form A in aqueous 2-Propanol (IPA)

### Example 18

A solution of sodium hydroxide (1.18g, 1eq.) in a mixture of water/IPA (80% v/v) (63ml) was added dropwise to a suspension of risedronic acid (8.35g) in a mixture of water/IPA (80% v/v) (354.5ml) at reflux temperature. The reaction mixture was heated at reflux temperature for 2 hours. The reaction mixture was cooled to room temperature and stirred at this temperature for 16 hours. Further cooling was performed using an ice-bath. The precipitate was then filtered, washed with IPA (1×20ml) and dried in a vacuum oven at 50°C for 24 hours to give 6.09g of sodium risedronate crystal form **A.**

### Preparation of Sodium Risedronate crystal form BB

### Example 19

A suspension of sodium risedronate (10.0g) in water (220 mL) was heated to 70°C and maintained at that temperature for two hours. Cold (0°C) IPA (1600 mL) was added, in one portion, to the hot solution to obtain a precipitate. The mixture was then cooled in an ice bath for 1.5 hours. The precipitate was then filtered, washed with IPA (2 x 30 mL), and dried in a vacuum oven at 50°C for 25 hours to yield 8.8 g (92%) of sodium risedronate for BB. The obtained risedronate sodium we stirred in IPA (150mL) at reflux temperature for 17 hours. The solid was then isolated by filtration, washed with IPA (2x17 mL) and dried in vacuo at 50°C for 27 hours to give 7.9g (90%) of sodium risedronate crystal form BB.

### Preparation of crystal form B1 in aqueous Ethanol

### Example 20

A solution of sodium hydroxide (2.77g, two equivalents) in a mixture of water/Ethanol (50% v/v) (30ml) was added dropwise to a suspension of Risedronic acid (10.0g) in a mixture of water/Ethanol (50% v/v) (170ml) at reflux temperature. The reaction mixture was held at reflux temperature for 24 hours. The reaction mixture was cooled to room temperature. Further cooling was performed using an ice-bath. The precipitate was collected by filtration, washed with Ethanol (1x10ml) and dried in a vacuum oven at 50°C for 24 hours to give 7.80g of sodium risedronate crystal form B1.

### Preparation of Mixtures of Forms B and B1

### Example 21

A solution of sodium hydroxide (1.18g,1 eq.) in a mixture of water/ethanol (24% v/v) (16.5ml) was added dropwise to a suspension of risedronic acid (8.35g) in a mixture of water/ethanol (24% v/v) (94ml) at reflux temperature. The reaction mixture was heated at reflux temperature for 19 hours. The reaction mixture was cooled to room temperature. Further cooling was performed using an ice-bath. The precipitate was then filtered, washed with ethanol (2×10ml) and dried in a vacuum oven at 50°C for 24 hours to give 8.84g (92%) of sodium risedronate mixture of crystal form **B1** and B (LOD by TGA=6.9%).

### Example 22

A solution of sodium hydroxide (1.18g, 1eq.) in a mixture of water/ethanol (6% v/v) (13.3ml) was added dropwise to a suspension of risedronic acid (8.35g) in a mixture of water/ethanol (6% v/v) (75.5ml) at reflux temperature. The reaction mixture was heated at reflux temperature for 18 hours. The reaction mixture was cooled to room temperature. Further cooling was performed using an ice-bath. The precipitate was then filtered, washed with ethanol (2x10ml) and dried in a vacuum oven at 50°C for 41 hours to give 8.96g (90%) of sodium risedronate mixture of crystal form **B1** and B (LOD by TGA=6.65%).

### Example 23

A solution of sodium hydroxide (1.18g, 1eq.) in a mixture of water/ethanol (11% v/v) (14ml) was added dropwise to a suspension of risedronic acid (8.35g) in a mixture of water/ethanol (11% v/v) (80ml) at reflux temperature. The reaction mixture was heated at reflux temperature for 19 hours. The reaction mixture was cooled to room temperature. Further cooling was performed using an ice-bath. The precipitate was then filtered, washed with ethanol (2x10ml) and dried in a vacuum oven at 50°C for 41 hours to give 8.67g of sodium risedronate mixture of crystal form **B1** and B.

### Example 24

A solution of sodium hydroxide (1.18g, 1eq.) in a mixture of water/ethanol (16% v/v) (15ml) was added dropwise to a suspension of risedronic acid (8.35g) in a mixture of water/ethanol (16% v/v) (84.5ml) at reflux temperature. The reaction mixture was heated at reflux temperature for 21 hours. The reaction mixture was cooled to room temperature. Further cooling was performed using an ice-bath. The precipitate was then filtered, washed with ethanol (2x10ml) and dried in a vacuum oven at 50°C for 24 hours to give 8.73g (90%) of sodium risedronate mixture of crystal form **B1** and B (LOD by TGA=7.1%).

### Example25

A solution of sodium hydroxide (1.18g, 1eq.) in a mixture of water/ethanol (20% v/v) (15.7ml) was added dropwise to a suspension of risedronic acid (8.35g) in a mixture of water/ethanol (20% v/v) (89ml) at reflux temperature. The reaction mixture was heated at reflux temperature for 20 hours. The reaction mixture was cooled to room temperature. Further cooling was performed using an ice-bath. The precipitate was then filtered, washed with ethanol (1x20ml) and dried in a vacuum oven at 50°C for 24 hours to give 8.75g of sodium risedronate mixture of crystal form **B1** and B.

### Preparation of crystal form D in Ethanol

### Example 26

1.23g (1 eq.) of sodium hydroxide were added to a suspension of risedronic acid (8.35g) in ethanol (834ml) at room temperature. The reaction mixture was heated to reflux temperature for 13 days. The reaction mixture was cooled to room temperature. Further cooling was performed using an ice-bath. The precipitate was then filtered, washed with ethanol (1x15ml) and dried in a vacuum oven at 50°C for 48 hours to give 7.28g of sodium risedronate crystal form **D**.

### Preparation of crystal forms by heat

### Example 27

About 100 mg of a mixture of risedronate forms B and A was kept in an open bottle in the oven at 160°C
5 hours, to yield form F.

### Example 28

About 100 mg risedronate mixture of forms A and E was kept in an open bottle in the oven at 160°C 5 hours, to yield form G.

### Example 29

About 100 mg risedronate form E was kept in an open bottle in the oven at 60°C 20 hours, to yield a mixture of form E and form A.

### Preparation of crystal forms by exposure at high relative humidity

### Example 30

About 100 mg risedronate form C was spread in a petri dish at a controlled relative humidity (±5%) of 80% for a period of one week, to yield a mixture of form C and form H.

### Example 31

About 100 mg risedronate form D was spread in a petri dish at a controlled relative humidity (±5%) of 80% for a period of one week, to yield a mixture of form D and form B.

### Example 32

About 100 mg risedronate form E was spread in a petri dish at a controlled relative humidity (±5%) of 80% for a period of one week, to yield form A with a small quantity of form D.

### Example 33

About 100 mg risedronate form F was spread in a petri dish at a controlled relative humidity (±5%) of 80% for a period of one week, to yield a mixture of form BB .

### Example 34

About 100 mg risedronate form F was spread in a petri dish at a controlled relative humidity (±5%) of 100% for a period of one week, to yield form BB.

### Example 35

About 100 mg risedronate form G was spread in a petri dish at a controlled relative humidity (±5%) of 100% for a period of one week, to yield a mixture of form G and form A.

**Table V**

| Summary of physical stability at high relative humidity (One week storage at relative humidities) | | | |
|---|---|---|---|
| Crystal Form | Relative humidity (%) | Resulting form | TGA Weight Loss (%) |
| A (LB-91 | 80 | A | 13.6 |
| B (LB-89) | 80 | B | 6.5 |
| C (LB-87) | 80 | H | 16.0 |
| D (MS-273) | 80 | D>B | 1.1 % |
| E (MS-277) | 80 | A | 14.0 |
| F (LB- 104 160C 8 hr) | 20-60 | F | 4-6 |
| | 80-100 | BB>A | 11.3-12.3 |
| G (MS-257 160C 8 hr) | 20-80 | G | 9.4-10.2 |
| | 100 | G+A | 12.6 |

### Polymorphic transformations by slurries in water, Ethanol and IPA Formation of crystal form B by slurry of Sodium Risedronate hemipentahydrate (A) in EtOH/water (1:1)

### Example 36

Sodium Risedronate hemipentahydrate (10.0g) was stirred in a mixture ofwater/Ethanol (1:1, 200ml). The suspension was heated at reflux temperature for 19 hours and then cooled to room temperature. The solid was filtered, washed with Ethanol (1x20ml) and dried in a vacuum oven at 50°C for 24 hours to give 8.78g of sodium risedronate crystal form B.
Form D to Form A (in water at reflux)

### Example 37

Sodium Risedronate crystal form D (3.0g) was stirred in water (30ml). The suspension was heated to reflux temperature to obtain a clear solution. Then the solution was cooled to room temperature. The resulting precipitate was filtered, washed with water (1x5ml), and dried in a vacuum oven at 50°C for 29 hours to give 1.58g of sodium risedronate crystal form A.

### Example 38

Sodium Risedronate crystal form D (2.0g) was stirred in water (30ml) at room temperature for 16 hours. The solid was then collected by filtration, washed with water (1 x 2ml) and dried in a vacuum oven at 50°C for 23 hours to give 0.25g of sodium risedronate crystal form A.

The mother-liquid was evaporated to dryness to give 1.30g of Sodium Risedronate crystal form B.

### Example 39

Sodium Risedronate crystal form B (2.0g) was stirred in water (20m1). The suspension was heated to reflux temperature to obtain a clear solution. Then the solution was cooled to room temperature. Further cooling was performed using an ice-bath. The resulting precipitate was filtered, washed with water (1× 10ml), and dried in a vacuum oven at 50°C for 72 hours to give 0.58g of sodium risedronate crystal form A.

### Example 40

Sodium Risedronate crystal form BB>A (2.0g) was stirred in water (25ml). The suspension was heated at reflux temperature for 18 hours to obtain a turbid solution and the turbid solution was cooled to room temperature. Further cooling was performed using an ice-bath. The resulting precipitate was filtered, washed with water (2x5ml) and dried in a vacuum oven at 50°C for 22 hours to give 0.22g of sodium risedronate crystal form A.

### Example 41

Sodium risedronate crystal form BB>A (3.0g) was stirred in Ethanol (45ml). The suspension was heated at reflux temperature for 18 hours to. The resulting solution was cooled to room temperature. The resulting precipitate was filtered, washed with water (1x15ml) and dried in a vacuum oven at 50°C for 26 hours to give 2.71 g of sodium risedronate crystal form D.

### Example 42

### Stability test of Risedronate tablets

A tablet containing Risedronate sodium active ingredient (about 12% w/w of the tablet) was kept in a securitainer at 40 degrees, 75% relative humidity for 6 months. The crystal form (detected from X-Ray powder diffraction data) of Risedronate active ingredient in the tablet are shown in the following table.

**Crystal form of Risedronate sodium active ingredient in a pharmaceutical tablet kept at 40°C. 75% RH**

| Time Interval | Crystal form |
|---|---|
| t=0 | B |
| 2 Weeks | B |
| 1 Month | B |
| 2 Months | B |
| 5 Months | B |
| *6 Months* | B |

### Example 43

### Stability test of Risedronate tablets

A tablet containing Risedronate sodium active ingredient (about 12% w/w of the tablet) was kept in a securitainer at ambient temperature for 4 months. The crystal form (detected from X-Ray powder diffraction data) of Risedronate active ingredient in the tablet are shown in the following table.

**Crystal form of Risedronate sodium active ingredient in a pharmaceutical tablet kept at ambient temperature**

| Time Interval | Crystal form |
|---|---|
| t = 0 | B |
| 4 Months | B |

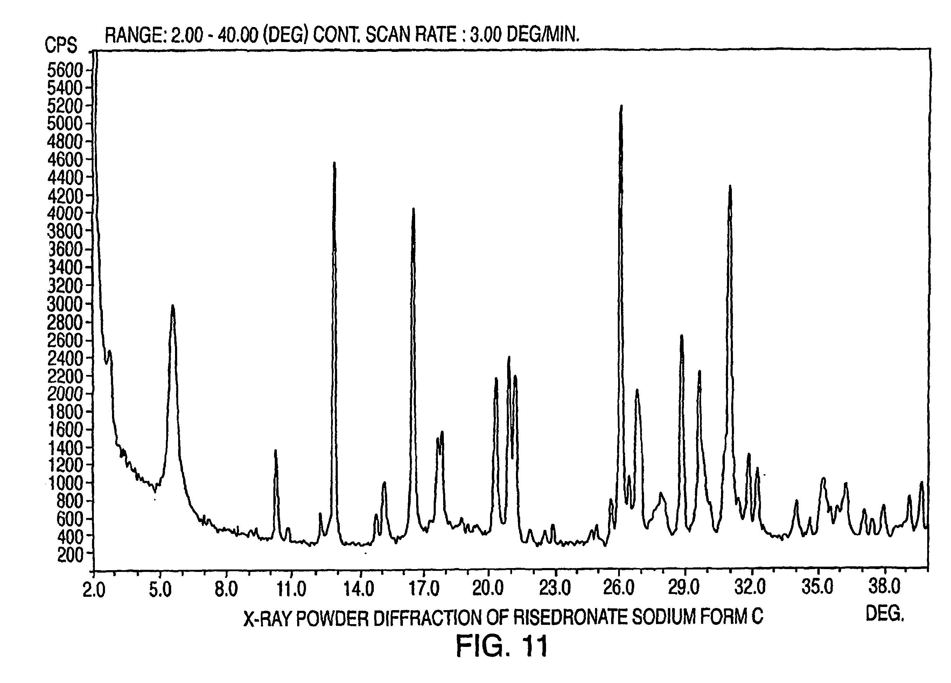

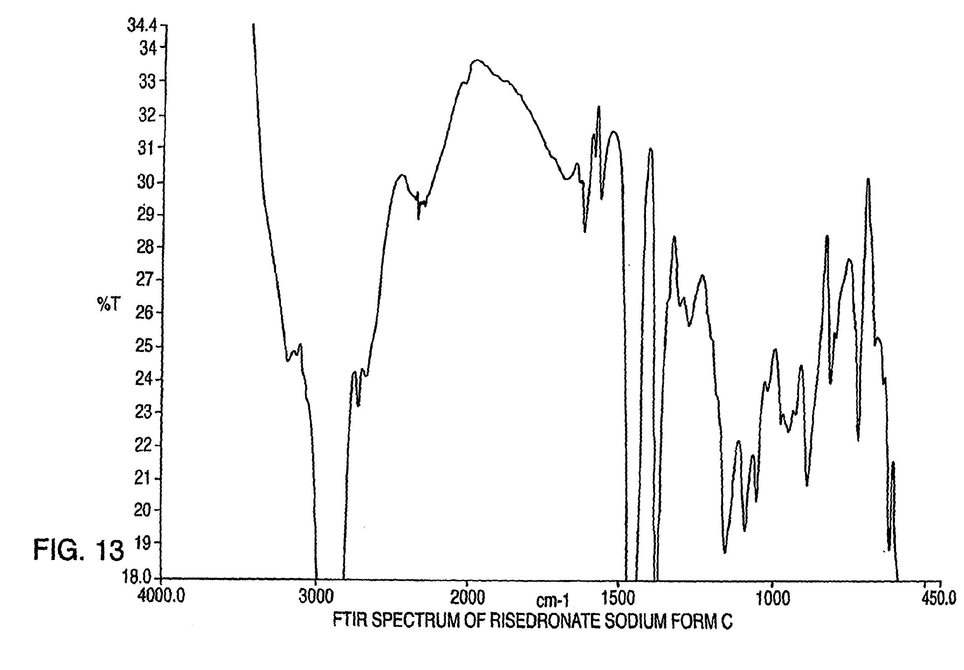

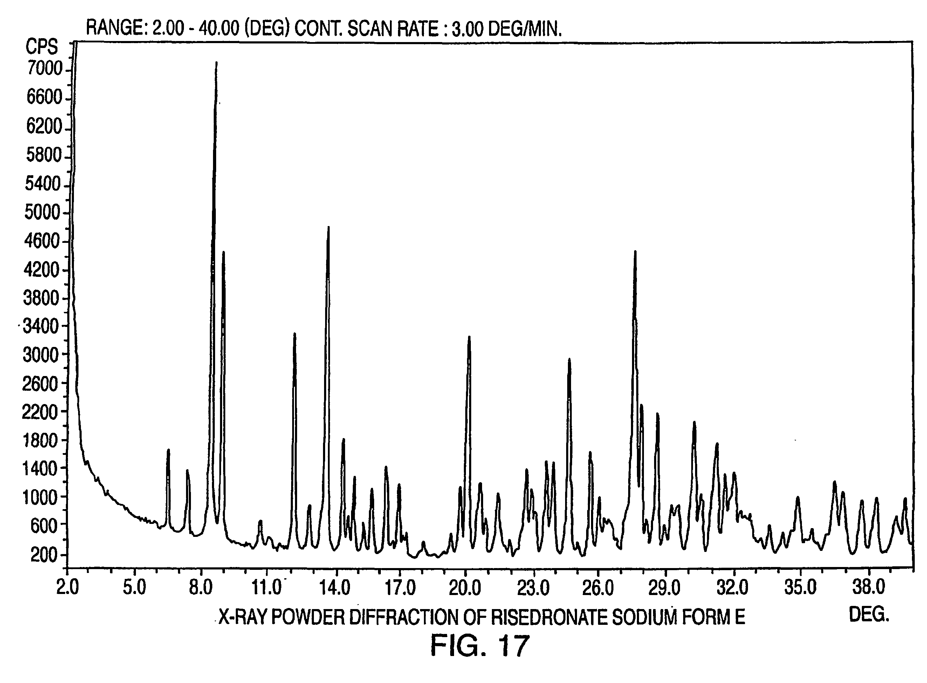

Further aspects and features of the present invention are set out in the following numbered clauses.
1. Risedronate sodium in crystalline form B characterized by X-ray peaks at 6.0, 14.4, 19.6, 24.9 and 25.4 degrees two-theta.
2. The risedronate sodium in crystalline form B of clause 1, that is pure form B, comprising less than about 1 weight % risedronate sodium in crystalline form A.
3. The substantially pure risedronate sodium in crystalline form B of clause 2, comprising less than about 0.5 weight % risedronate sodium in crystalline form A.
4. The substantially pure risedronate sodium in crystalline form B of clause 2, stable against transformation to risedronate sodium in crystalline form A.
5. The Risedronate sodium of clause 4 wherein less than about 20% of form B is converted to form A during storage for two years at room temperature.
6. Risedronate sodium in crystalline form B stable against transformation to risedronate sodium in crystalline form A when exposed to 75% RH at 40° C for at least about three months.
7. The risedronate sodium form B of clause 6 stable against transformation to risedronate sodium in crystalline form A when exposed to 75% RH at 40°C for at least about 5 months.
8. The risedronate sodium form B of clause 7 stable against transformation to risedronate sodium in crystalline form A when exposed to 75% RH at 40°C for at least about 6 months.
9. Risedronate sodium form B wherein not more that about 20% by weight of the form B transforms to form A when exposed to 75% RH at 40°C for at least 3 months.
10. The risedronate sodium form B of clause 9 wherein not more than about 10% by weight of the form B transforms to form A when exposed to 75% RH at 40°C for at least 3 months.
11. The risedronate sodium form B of clause 10 wherein not more than about 5% by weight of the form B transforms to form A when exposed to 75% RH at 40° C for at least 3 months.
12. Risedronate sodium in a crystalline form characterized by x-ray diffraction peaks at 6.0, 14.4, 19.6, 24.9 and 25.4 degrees two-theta.
13. The risedronate sodium of clause 12 characterized by an x-ray diffraction diagram substantially as shown in figure 4.
14. Risedronate sodium in a crystalline form characterized by FTIR absorption bands at 624, 951, 796, 912, 931, 1046, 1105, 1123, 1373, and 1641 cm⁻¹.
15. The risedronate sodium of clause 14 characterized by a FTIR spectrum substantially as shown in figure 6.
16. A method for preparing risedronate sodium having at least one characteristic of form B comprising the steps of:
   refluxing a mixture of risedronic acid, a sodium base, and a liquid comprising about 40% to about 60% v/v lower alkanol in water, and
   isolating risedronate sodium having at least one characteristic of form B from the resulting mixture.
17. The method of clause 16 further comprising the step of, before isolating risedronate sodium having at least one characteristic of form B, cooling the mixture.
18. The method of clause 17 wherein the cooling is to a temperature of about room temperature.
19. The method of clause 17 wherein the cooling is to a temperature of about 5° C or less.
20. The method of clause 16 wherein the molar ratio between risedronic acid and the sodium base is between about 1:0.8 and about 1:1.2.
21. Method of clause 20 wherein the molar ratio between risedronic acid and the sodium base is between about 1:1 and about 1:1.2.
22. The method of clause 20 wherein the sodium base is sodium hydroxide.
23. The method of clause 16 wherein the lower alkanol is selected from the group consisting of methanol, ethanol and isopropanol.
24. The method of clause 16 wherein the liquid consists essentially of a mixture of about 50% ethanol and about 50%, v/v, water.
25. The method of clause 16 wherein the liquid consists essentially of a mixture of about 50% methanol and about 50% water, v/v.
26. The method of clause 16 wherein the risedronate sodium having at least one characteristic of form B is pure form B.
27. The method of clause 16 wherein the risedronate sodium having at least one characteristic of form B is stable form B.
28. A method for preparing risedronate sodium having at least one characteristic of form B comprising the steps of:
   refluxing a mixture of risedronic acid, a sodium base, and a liquid comprising about 20% to about 70%, v/v, methanol in water, and
   isolating risedronate having at least one characteristic of form B from the resulting mixture.
29. The method of clause 28 further comprising the step of, prior to isolating risedronate sodium having at least one characteristic of form B, cooling the mixture.
30. The method of clause 29 wherein the cooling is to a temperature of about room temperature.
31. The method of clause 29 wherein the cooling is to a temperature of about 5°C or less.
32. The method of clause 28 wherein the molar ratio between risedronic acid and the sodium base is between about 1:0.8 and about 1:1.2.
33. The method of clause 32 wherein the molar ratio between risedronic acid and the sodium base is between about 1:1 and about 1: 1.2.
34. The method of clause 28 wherein the sodium base is sodium hydroxide.
35. The method of clause 28 wherein the risedronate sodium having at least one characteristic of form B is pure form B.
36. The method of clause 28 wherein the risedronate sodium having at least one characteristic of form B is stable form B.
37. Risedronate sodium in a crystalline form characterized by x-ray peaks at about 8.5, 9.1, 9.5, and 12.2 +/- 0.2 degrees two-theta.
38. The risedronate sodium of clause 37 characterized by additional x-ray peaks at about 14.3, 16.9, 19.7, 23.5, 28.8, and 33.6 +/- 0.2 degrees two-theta.
39. The risedronate sodium of clause 38 characterized by an x-ray diffraction diagram substantially as shown in figure 7.
40. Risedronate sodium in form BB.
41. A method of making risedronate sodium having at least one characteristic of form BB comprising the steps of:
   a) providing a solution of risedronate sodium in water at a temperature of about 70°C or more,
   b) adding iso-propanol to the solution to obtain a solid-in-liquid suspension,
   c) isolating the solid from the suspension,
   d) refluxing the isolated solid in suspension in iso-propanol for at least about 10 hours, and
   e) isolating risedronate sodium having at least one characteristic of form BB from the suspension.
42. Risedronate sodium having at least one characteristic of form BB made by the method of clause 41.
43. A method of making risedronate sodium having at least one characteristic of form BB comprising the step of exposing risedronate sodium having at least one characteristic of form F to an atmosphere having a relative humidity of at least about 80%.
44. The method of clause 43 wherein the risedronate sodium having at least one characteristic of form F is exposed for a period of time of at least about 1 week.
45. Risedronate sodium having at least one characteristic of form BB made by the method of clause 43.
46. Risedronate disodium salt in a crystalline form characterized by x-ray diffraction peaks at about 6.5, 14.7, 21.2, 27.7, and 32.4 +/- 0.2 degrees two-theta.
47. The risedronate disodium salt of clause 46 having an x-ray diffraction diagram substantially as shown in figure 9.
48. A method of making risedronate sodium in crystalline form B in admixture with crystalline form B 1 comprising the steps of:
   refluxing a combination of risedronic acid and a sodium base in a liquid comprising between about 5% and about 25%, v/v, ethanol, the remainder consisting essentially of water, and
   isolating the risedronate sodium form B from the combination.
49. The method of clause 48 further comprising the step, prior to the isolation step, of cooling the combination.
50. The method of clause 49 wherein the cooling is to a temperature of about room temperature.
51. The method of clause 49 wherein the cooling is to a temperature of about 5°C or less.
52. The method of clause 48 wherein the sodium base is sodium hydroxide.
53. A method of making risedronate disodium in a crystalline form having at least one characteristic of form B comprising the steps of;
   refluxing a mixture of risedronic acid, at least about two equivalents of a sodium base, and a liquid comprising water and ethanol, 50/50 v/v, and
   isolating risedronate disodium having at least one characteristic of form B1 from the mixture.
54. The method of clause 53 further comprising the step, before the isolating step, of cooling the combination.
55. The method of clause 54 wherein the cooling is to a temperature of about room temperature.
56. The method of clause 54 wherein the cooling is to a temperature of about 5°C or less.
57. The method of clause 53 wherein the sodium base is sodium hydroxide.
58. Risedronate sodium having at least one characteristic of form B1 made by the method of clause 52.
59. Risedronate sodium in a crystalline form characterized by X-ray peaks at about 5.6, 10.3, 12.9, 26.5, and 30.9° +/- 0.2 degrees two-theta.
60. The risedronate sodium of clause 59 characterized by an x-ray diffraction diagram substantially as shown in figure 11.
61. Risedronate sodium in a crystalline form characterized by absorption bands at about 615, 666, 1089, 1563 and 1615 cm-1 in FTIR spectroscopy.
62. The risedronate sodium of clause 61 characterized by an FTIR spectrum substantially as shown in figure 13.
63. A method of making risedronate sodium having at least one characteristic of form C comprising the step of refluxing a mixture of risedronic acid, a sodium base, and a liquid comprising about 3%, v/v, ethanol, the remainder of the mixture by volume consisting essentially of water.
64. The method of clause 63 further comprising the steps of:
   cooling the mixture to about room temperature,
   isolating risedronate sodium having at least one characteristic of form C.
65. The method of clause 64 further comprising the step of, before isolating risedronate sodium having at least one characteristic of form C, further cooling the mixture to a temperature of about 5° C or less.
66. The method of clause 63 wherein the sodium base is sodium hydroxide.
67. Risedronate sodium having at least one characteristic of form C made by the method of clause 63.
68. Risedronate sodium in a crystalline form characterized by x-ray diffraction peaks at about 9.9, 17.2, 22.1, 27.9, and 29.2 +/- 0.2 degrees two-theta.
69. The risedronate sodium of clause 68 characterized by an x-ray diffraction diagram substantially as shown in figure 14.
70. Risedronate sodium in a crystalline form characterized by absorption bands at 697, 807, 854, 955, 1187, 1218, 1576, 1646, and 1719 cm⁻¹ in FTIR spectroscopy.
71. The risedronate sodium of clause 70 characterized by an FTIR spectrum substantially as shown in figure 16.
72. A method of making risedronate sodium having at least one characteristic of form D comprising the step of refluxing a mixture of risedronic acid, a sodium base, and a liquid that is selected from the group consisting of ethanol, methanol, and mixtures of methanol and water having up to about 11 %, v/v, water.
73. The method of clause 72 further comprising the steps of:
   cooling the mixture to a temperature of about room temperature, and
   isolating risedronate sodium having at least one characteristic form D.
74. The method of clause 73 further comprising the step of, prior to isolating risedronate sodium having at least one characteristic of form D, further cooling the mixture to a temperature of about 5° C or less.
75. Risedronate sodium having at least one characteristic of form D made by the method of clause 72.
76. A method of making risedronate sodium comprising crystalline form B comprising the step of exposing risedronate sodium crystalline form D to an atmosphere of 80% to 100% relative humidity.
77. The method of clause 76 wherein the risedronate sodium having at least one characteristic form B is pure form B.
78. Risedronate sodium in a crystalline form characterized by x-ray peaks at about 8.4, 8.9, 13.6, 27.6, and 27.9 +/- 0.2 degrees two-theta.
79. The method of clause 76 wherein the risedronate sodium having at least one characteristic of form B is stable form B.
80. The risedronate sodium of clause 79 characterized by an x-ray diffraction diagram substantially as shown in figure 17.
81. Risedronate sodium in crystalline a crystalline form characterized by absorption bands at 801, 890, 935, 1656, and 1689 cm⁻¹ in FTIR spectroscopy.
82. The risedronate sodium of clause 81 characterized by an FTIR spectrum substantially as shown in figure 19.
83. A method of making risedronate sodium having at least one characteristic of form E comprising the step of refluxing a combination of risedronic acid, a sodium base, and a liquid comprising 80%, v/v water, the remainder being methanol.
84. The method of clause 83 further comprising the steps of:
   cooling the combination to a temperature of about room temperature,
   isolating risedronate sodium having at least one characteristic of form E.
85. The method of clause 84 further comprising the step of, before isolating the risedronate sodium having at least one characteristic of form E, cooling the mixture to a temperature of about 5° C or less.
86. The method of clause 83 wherein the sodium base is sodium hydroxide.
87. Risedronate sodium having at least one characteristic of form E made by the method of clause 83.
88. A method of making risedronate sodium comprising crystalline form E comprising the steps of:
   a) refluxing a combination of risedronic acid, sodium hydroxide, and water,
   b) cooling the combination to a temperature of about room temperature, and
   c) isolating risedronate sodium comprising form E from the combination.
89. The method of clause 88 further comprising the step, before step c, of further cooling the combination to a temperature of about 5° C or less.
90. Risedronate sodium in a crystalline form characterized by x-ray peaks at about 6.6, 8.4, 8.9, 12.2, and 18.6 +/- 0.2 degrees two-theta.
91. The risedronate sodium of clause 90 characterized by an x-ray diffraction diagram substantially as shown in figure 20.
92. Risedronate sodium in a crystalline form characterized by absorption bands at 971, 1133, and 1306 cm⁻¹ in FTIR spectroscopy.
93. The risedronate sodium of clause 92 characterized by an FTIR spectrum substantially as shown in figure 22.
94. Risedronate sodium having at least one characteristic of form F stable against transformation to form A in an atmosphere having a relative humidity up to 60%.
95. A method of making risedronate sodium having at least one characteristic of form F comprising the step of heating risedronate sodium form A, form B, or a mixture of these at 160°C_{.}
96. Risedronate sodium having at least one characteristic of form F made by the method of clause 95.
97. Risedronate sodium in a crystalline form characterized by x-ray peaks at about 8.0, 9.9, 12.2, 15.2, and 19.6 +/- 0.2 degrees two-theta.
98. The risedronate sodium of clause 97 characterized by an x-ray diffraction diagram substantially as shown in figure 23.
99. Risedronate sodium in a crystalline form characterized by absorption bands at 724, 871, 1174, and 1285 cm⁻¹ in FTIR spectroscopy.
100. The risedronate sodium of clause 99 characterized by an FTIR spectrum substantially as shown in figure 25.
101. A method of making risedronate sodium having at least one characteristic of form G comprising the step of heating a mixture of risedronate sodium forms A and E at a temperature between about 120° and about 180° C.
102. Risedronate sodium having at least one characteristic of form G made by the method of clause 101.
103. Risedronate sodium in a crystalline form characterized by x-ray diffraction reflections at about 6.9, 9.8, 10.9, 13.7, 16.0, and 18.0 +/- 0.2 degrees two theta.
104. A method for making risedronate sodium having at least one characteristic of form H comprising the step of exposing risedronate sodium form C in an atmosphere having a relative humidity between about 60% and about 100%.
105. The method of clause 104 wherein the atmosphere has a relative humidity of at least about 80%.
106. Risedronate sodium having at least one characteristic of form H made by the method of clause 104.
107. A method of making risedronate sodium form B comprising the steps of:
   a) combining a solution of sodium hydroxide in a mixture of water and ethanol, 60% v/v, with risedronic acid, wherein the amount of sodium hydroxide combined is about one equivalent based on risedronic acid,
   b) heating the combination at reflux for a reflux time,
   c) cooling the mixture to about room temperature,
   d) further cooling the mixture to a temperature of about 5° C or less, and
   e) isolating risedronate sodium form B.
108. The method of clause 107 wherein the risedronate sodium form B is risedronate sodium pure form B.
109. The method of clause 107 wherein the risedronate sodium form B is risedronate sodium stable form B.
110. A method of making risedronate sodium having at least one characteristic of form A comprising the step of refluxing a mixture of risedronic acid, a sodium base, and a liquid comprising about 20%, v/v, iso-propanol and water.
111. The method of clause 110 further comprising the steps of:
   cooling the mixture to a temperature of about room temperature, and
   isolating risedronate sodium having at least one characteristic form A.
112. The method of clause 110 further comprising the step of, before isolating risedronate sodium having at least one characteristic of form A, further cooling the mixture to a temperature of about 5°C or less.
113. A method of making risedronate sodium having at least one characteristic of form A comprising the steps of:
   a) refluxing a water solution of sodium risedronate,
   b) cooling the solution to obtain a suspension, and
   c) isolating risedronate sodium having at least one characteristic of form A from the suspension.
114. A method of making risedronate sodium form A comprising the step of exposing risedronate sodium form E, risedronate sodium form G, or a mixture of risedronate sodium forms E and G to an atmosphere having a relative humidity of at least about 80% for a period of about one week.
115. A method of making risedronate sodium form A comprising the step of incubating risedronate sodium form E at 60° C for a period of at least about 5 hours.
116. A method of making risedronate sodium form A comprising the step of treating risedronate sodium form B or form D, or a mixture of these, with water at a temperature between about room temperature and about reflux temperature.
117. The method of clause 116 wherein the risedronate sodium is risedronate sodium form B.
118. The method of clause 116 wherein the risedronate sodium is risedronate sodium form D.
119. A method of making risedronate sodium having at least one characteristic form B comprising the step of treating risedronate sodium form A with 1:1, v/v, mixture of a lower alkanol and water at a temperature between about room temperature and about reflux temperature.
120. The method of clause 119 wherein the risedronate sodium having at least one characteristic of form B is risedronate sodium pure form B.
121. The method of clause 119 wherein the lower alkanol is ethanol.
122. A method of making risedronate sodium having at least one characteristic of form D comprising the step of treating risedronate sodium comprising form BB in ethanol at a temperature between about room temperature and about reflux temperature.
123. Risedronate sodium having at least one characteristic of form D made by the method of clause 122.
124. A pharmaceutical composition comprising pure risedroante sodium form B and at least one pharmaceutically acceptable excipient.
125. The pharmaceutical composition of clause 124 wherein at least about 60% by weight of the risedronate sodium form B remains untransformed to form A when the composition is stored for at least 3 months at 40°C and 75% RH.
126. The pharmaceutical composition of clause 125 wherein at least about 60% by weight of the risedronate sodium form B remains untransformed to form A when the composition is stored for at least 6 months at 40°C and 75% RH.
127. The pharmaceutical composition of clause 125 wherein at least about 60% by weight of the risedronate sodium form B remains untransformed to form A when the composition is stored for at least about 2 years at a temperature of about 25°C and 75% RH. 6 months at 40°C and 75% RH.
128. The pharmaceutical composition of clause 125 in the form of a tablet.
129. A pharmaceutical composition comprising stable risedronate sodium form B and at least one pharmaceutically acceptable excipient.
130. A pharmaceutical composition comprising risedronate sodium crystalline form C and at least one pharmaceutically acceptable excipient.
131. A pharmaceutical composition comprising risedronate sodium crystalline form B1 and at least on pharmaceutically acceptable excipient.
132. A pharmaceutical composition comprising risedronate sodium crystalline form D and at least one pharmaceutically acceptable excipient.
133. A pharmaceutical composition comprising risedronate sodium crystalline form BB and at least one pharmaceutically acceptable excipient.
134. A pharmaceutical composition comprising risedronate sodium crystalline form E and at least one pharmaceutically acceptable excipient.
135. A pharmaceutical composition comprising risedronate sodium crystalline form F and at least one pharmaceutically acceptable excipient.
136. A pharmaceutical composition comprising risedronate sodium crystalline form G and at least one pharmaceutically acceptable excipient.
137. A pharmaceutical composition comprising risedronate sodium crystalline form H and at least one pharmaceutically acceptable excipient.
138. A method of treating osteoporosis comprising the step of administering to a patient suffering from osteoporosis an osteoporosis treating effective amount of risedronate sodium pure form B.
139. A method of treating osteoporosis comprising the step of administering to a patient suffering from osteoporosis an osteoporosis treating effective amount of at least one of risedronate sodium crystal forms B, C, D, B1, BB, E, F, G, and H.

## Claims

1. Risedronate sodium in crystalline form B **characterized by** X-ray peaks at 6.0, 14.4, 19.6, 24.9 and 25.4 degrees two-theta, comprising less than about 1 weight % risedronate sodium in crystalline form A.

2. The risedronate sodium in crystalline form B of claim 1, comprising less than about 0.5 weight % risedronate sodium in crystalline form A.

3. The risedronate sodium in crystalline form B of claim 1, wherein the form B is stable against transformation to risedronate sodium in crystalline form A.

4. The risedronate sodium in crystalline form B of claim 3, wherein less than about 20% of form B is converted to form A during storage for two years at room temperature.

5. The risedronate sodium in crystalline form B of claim 3, wherein the form B is stable against transformation to risedronate sodium in crystalline form A when exposed to 75% RH at 40°C for at least about three months, preferably for at least about 5 months, more preferably for at least about 6 months.

6. The risedronate sodium in crystalline form B of claim 1, wherein not more than about 20% by weight of the form B transforms to form A when exposed to 75% RH at 40°C for at least 3 months.

7. The risedronate sodium in crystalline form B of claim 6, wherein not more than about 10% by weight of the form B transforms to form A when exposed to 75% RH at 40°C for at least 3 months.

8. The risedronate sodium in crystalline form B of claim 7 wherein not more than about 5% by weight of the form B transforms to form A when exposed to 75% RH at 40°C for at least 3 months.

9. A method for preparing risedronate sodium having at least one characteristic of form B comprising the steps of :
refluxing a mixture of risedronic acid, a sodium base, and a liquid comprising about 40% to about 60% v/v lower alkanol in water, and
isolating risedronate sodium having at least one characteristic of form B from the resulting mixture.

10. The method of claim 9 further comprising the step of, before isolating risedronate sodium having at least one characteristic of form B, cooling the mixture.

11. The method of claim 10 wherein the cooling is to a temperature of about room temperature.

12. The method of claim 10 wherein the cooling is to a temperature of about 5°C or less.

13. The method of any one of claims 9 to 12 wherein the molar ratio between risedronic acid and the sodium base is between about 1:0.8 and about 1:1.2.

14. The method of claim 13 wherein the molar ratio between risedronic acid and the sodium base is between about 1:1 and about 1:1.2.

15. The method of any one of claims 9 to 14, wherein the sodium base is sodium hydroxide.

16. The method of any one of claims 9 to 15, wherein the lower alkanol is selected from the group consisting of methanol, ethanol and isopropanol.

17. The method of any one of claims 9 to 15, wherein the liquid consists essentially of a mixture of about 50% ethanol and about 50%, v/v, water.

18. The method of any one of claims 9 to 15, wherein the liquid consists essentially of a mixture of about 50% methanol and about 50% water, v/v.

19. The method of any one of claims 9 to 18, wherein the risedronate sodium having at least one characteristic of form B is pure form B.

20. The method of any one of claims 9 to 19, wherein the risedronate sodium having at least one characteristic of form B is stable form B.

21. A method for preparing risedronate sodium having at least one characteristic of form B comprising the steps of :
refluxing a mixture of risedronic acid, a sodium base, and a liquid comprising about 20% to about 70%, v/v, methanol in water, and
isolating risedronate having at least one characteristic of form B from the resulting mixture.

22. The method of claim 21 further comprising the step of, prior to isolating risedronate sodium having at least one characteristic of form B, cooling the mixture.

23. The method of claim 22 wherein the cooling is to a temperature of about room temperature.

24. The method of claim 22 wherein the cooling is to a temperature of about 5°C or less.

25. The method of any one of claims 21 to 24, wherein the molar ratio between risedronic acid and the sodium base is between about 1:0.8 and about 1:1.2.

26. The method of claim 25 wherein the molar ratio between risedronic acid and the sodium base is between about 1:1 and about 1:1.2.

27. The method of any one of claims 21 to 26, wherein the sodium base is sodium hydroxide.

28. The method of any one of claims 21 to 27 wherein the risedronate sodium having at least one characteristic of form B is pure form B.

29. The method of any one of claims 21 to 28, wherein the risedronate sodium having at least one characteristic of form B is stable form B.

30. A method of making risedronate sodium in crystalline form B in admixture with crystalline form B1 comprising the steps of:
refluxing a combination of risedronic acid and a sodium base in a liquid comprising between about 5% and about 25%, v/v, ethanol, the remainder consisting essentially of water, and
isolating the risedronate sodium form B from the combination.

31. The method of claim 30 further comprising the step, prior to the isolation step, of cooling the combination.

32. The method of claim 31 wherein the cooling is to a temperature of about room temperature.

33. The method of claim 31 wherein the cooling is to a temperature of about 5°C or less.

34. The method of any one of claims 30 to 33, wherein the sodium base is sodium hydroxide.

35. A method of making risedronate sodium comprising crystalline form B comprising the step of exposing risedronate sodium crystalline form D to an atmosphere of 80% to 100% relative humidity.

36. The method of claim 35 wherein the risedronate sodium having at least one characteristic form B is pure form B.

37. The method of claim 35 or claim 36 wherein the risedronate sodium having at least one characteristic of form B is stable form B.

38. A method of making risedronate sodium form B comprising the steps of :
a) combining a solution of sodium hydroxide in a mixture of water and ethanol, 60% v/v, with risedronic acid, wherein the amount of sodium hydroxide combined is about one equivalent based on risedronic acid,
b) heating the combination at reflux for a reflux time,
c) cooling the mixture to about room temperature,
d) further cooling the mixture to a temperature of about 5°C or less, and
e) isolating risedronate sodium form B.

39. The method of claim 38 wherein the risedronate sodium form B is risedronate sodium pure form B.

40. The method of claim 38 or claim 39 wherein the risedronate sodium form B is risedronate sodium stable form B.

41. A method of making risedronate sodium having at least one characteristic form B comprising the step of treating risedronate sodium form A with 1:1, v/v, mixture of a lower alkanol and water at a temperature between about room temperature and about reflux temperature.

42. The method of claim 41, wherein the risedronate sodium having at least one characteristic of form B is risedronate sodium pure form B.

43. The method of claim 41 or claim 42, wherein the lower alkanol is ethanol.

44. A pharmaceutical composition comprising pure risedroante sodium form B and at least one pharmaceutically acceptable excipient.

45. A pharmaceutical composition comprising risedroante sodium form B as defined in any one of claims 1 to 8, and at least one pharmaceutically acceptable excipient.

46. The pharmaceutical composition of claim 44 or claim 45 in the form of a tablet.

47. A pharmaceutical composition comprising stable risedronate sodium form B and at least one pharmaceutically acceptable excipient.

48. Risedronate sodium in crystalline form B as defined in any one of claims 1 to 8 for use in the treatment of osteoporosis.

49. Use of risedronate sodium in crystalline form B as defined in any one of claims 1 to 8 in the manufacture of a medicament for the treatment of osteoporosis.
